# FASCICULE DE BREVET EUROPEEN

(11) **EP 1 345 919 B1**
(45) Date de publication et mention de la délivrance du brevet: **22.05.2013**
(21) Numéro de dépôt: 01995766.1
(22) Date de dépôt: 21.12.2001
(51) Int. Cl.: C07D 309/10

(54) **NOUVEAU DERIVES C-GLYCOSIDES ET UTILISATION**
C-GLYKOSIDDERIVATE UND IHRE VERWENDUNG
NOVEL C-GLYCOSIDE DERIVATIVES AND USE THEREOF

(30) Priorité: 22.12.2000 FR 0016997
(43) Date de publication de la demande: 24.09.2003
(73) Titulaire: L'Oréal, 75008 Paris (FR)
(72) Inventeur: DALKO, Maria, F-91190 Gif sur Yvette (FR); BRETON, Lionel, F-78000 Versailles (FR)
(74) Mandataire: Le Blainvaux Bellegarde, Françoise
(86) Numéro de dépôt international: PCT/FR2001/004166
(87) Numéro de publication internationale: WO 2002/051828

(56) Documents cités:
- FR-A- 2 770 776
- US-A- 4 446 312
- F. RODRIGUES ET AL.: "A convenient, one-step, synthesis of beta-C-glycosidic ketones in aqueous media" CHEM. COMMUN., 2000, pages 2049-2050, XP002174157
- B. SCHÖNENBERGER ET AL.: "229. Enantioselective synthesis of pseudomonic acids. I. Synthesis of key intermediates" HELVETICA CHIMICA ACTA, vol. 65, 1982, pages 2333-2337, XP002174204
- M.G. HOFFMANN, R.R. SCHMIDT: "Reaktionen von Glycosyl-trichloracetimidaten mit silylierten C-Nucleophilen" LIEBIGS ANN. CHEM., 1985, pages 2403-2419, XP002174264
- J.M. BEMILLER ET AL.: "N-Substituted (beta-D-galactopyranosylmethyl)amines, and C-beta-D-galactopyranosylformamides, and related compounds" CARBOHYDRATE RESEARCH, vol. 200, 1990, pages 111-126, XP002174205
- P.G. GOEKJIAN ET AL.: "Preferred conformation of C-glycosides. 6. Conformational similarity of glycosides and corresponding C-glycosides" J. ORG. CHEM., vol. 56, 1991, pages 6412-6422, XP002174206

## Description

La présente invention se rapporte à de nouveaux dérivés C-glycosides, leur procédé de synthèse et les compositions les comprenant.

Un autre objet de l'invention se rapporte l'utilisation, dans un milieu physiologiquement acceptable, dans une composition cosmétique ou pour la préparation d'une composition pharmaceutique, d'au moins un dérivé C-glycoside, le composé ou la composition étant destinés à stimuler la synthèse des glycosaminoglycannes contenant un résidu D-glucosamine et/ou N-acétyl-D-glucosamine, avantageusement de l'acide hyaluronique, et/ou des protéoglycannes, avantageusement des protéoglycannes contenant de l'acide hyaluronique, par les fibroblastes et/ou les kératinocytes.

L'invention se rapporte également à un procédé de traitement cosmétique utilisant une telle composition cosmétique.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire. On y trouve aussi des leucocytes, des mastocytes ou encore des macrophages tissulaires. Il est également constitué de vaisseaux sanguins et de fibres nerveuses.

La matrice extracellulaire du derme, comme celle de tous les tissus conjonctifs de l'organisme, est composée de protéines appartenant plusieurs grandes familles : les collagènes, les glycoprotéines matricielles autres que les collagènes (fibronéctine, laminine), l'élastine et les protéoglycannes. On trouve également dans la matrice extracellulaire du derme, comme celle de tous les tissus conjonctifs de l'organisme, des glycosaminoglycannes sous forme libre (c'est à dire non liés à une protéine).

Il est maintenant bien établi que des interactions spécifiques existent entre ces différentes classes de protéines pour donner naissance à un tissu fonctionnel.

Les protéoglycannes sont des macromolécules complexes constituées d'un tronc protéique central ramifié, ou réseau de protéines, auquel sont attachés de très nombreuses chaînes latérales polyosidiques appelées glycosaminoglycannes.

Dans la suite de la présente demande, on désignera les protéoglycannes par l'abréviation PGs et les glycosaminoglycannes par l'abréviation GAGs.

Les GAGs ont longtemps été désignés sous le terme de mucopolysaccharides acides en raison de leur forte capacité de rétention d'eau, de leur nature glucidique et de leur caractère acide provenant de leurs multiples charges négatives.

Ainsi, la polarité des GAGs les, fait implicitement participer à certaines fonctions biologiques comme l'hydratation des tissus, la fixation des cations ou le rôle de barrière de filtration ionique.

Les PGs et les GAGs sont synthétisés par différentes cellules au niveau du derme et de l'épiderme: fibroblastes, kératinocytes et mélanocytes.

Les fibroblastes synthétisent majoritairement des collagènes, des glycoprotéines matricielles autres que les collagènes (fibronéctine, laminine), des protéoglycannes et de l'élastine. Les kératinocytes synthétisent majoritairement des GAGs sulfatés et de l'acide hyaluronique alors que les mélanocytes ne produisent apparemment pas d'acide hyaluronique.

Lorsqu'ils sont incorporés dans un PG, les GAGs sont des chaînes linéaires composées de la répétition d'un diholoside de base contenant toujours une hexosamine (glucosamine ou galactosamine) et un autre ose (acide glucuronique, acide iduronique ou galactose). La glucosamine est soit N-sulfatée, soit N-acétylée. En revanche, la galactosamine est toujours N-acétylée. En outre, il peut y avoir des sulfates O-liés sur l'hexosamine, l'acide uronique et le galactose.

Le fort caractère anionique des GAGs s'explique par la présence de groupes carboxylates au sein des acides hexuroniques (acide glucuronique et acide iduronique) et de groupes sulfate O- et N-liés.

Les principaux GAGs sont l'acide hyaluronique ou hyaluronanne (HA), l'héparane sulfate (HS), l'héparine (HP), la chondroïtine, la chondroïtine sulfate (CS), la chondroïtine 4-sulfate ou chondroïtine sulfate A (CSA), la chondroïtine 6-sulfate ou chondroïtine sulfate C (CSC), le dermatane sulfate ou chondroïtine sulfate B (CSB) et le kératane sulfate (KS) qui diffère des autres glycosaminoglycannes par la présence de galactose à la place de l'acide uronique.

Lorsqu'ils sont associés à une protéine sous forme de PG les GAGs sont liés par des structures d'ancrage aux différentes chaînes polypeptidiques, nommées protéine « core » ou protéine porteuse, et forment ainsi des molécules de PGs.

Les GAGs peuvent également exister dans la matrice extracellulaire sous forme libre c'est à dire non liée à une protéine matricielle : c'est notamment le cas de l'acide hyaluronique.

Lors de la synthèse des PGs, les GAGs sont polymérisés à partir de ces structures d'ancrage.

La synthèse des GAGs nécessite l'action coordonnée et concertée d'enzymes très spécifiques (transférases, épimérases, sulfotransférases) adjacentes dans la membrane du réticulum endoplasmique et de l'appareil de golgi. Puis une multitude de réactions biochimiques (N-désacétylation, N- et O-sulfatations, épimérisation) modifient les deux oses constitutifs du motif de base et cela de manière hétérogène le long de la chaîne. D'une chaîne d'héparane sulfate à l'autre par exemple, le rapport acide glucuronique/acide iduronique, la nature, le nombre et la position des O-sulfatations, ainsi que le rapport N-sulfate/O-sulfate peuvent varier, ce qui, potentiellement offre une immense diversité structurale.

De façon générale, les rôles biologiques des PGs sont très diversifiés, allant d'une fonction passive de support mécanique (par exemple serglycines) ou d'un rôle de barrière ionique de filtration moléculaire (par exemple perlecanne et bamacanne de la membrane basale glomérulaire), à des effets plus spécifiques dans l'adhésion, l'étalement, la prolifération, la différenciation cellulaire ou la morphogenèse, ou, à des effets très spécifiques d'interactions PG-protéine, tels que la fonction de récepteur du bétaglycanne ou l'interaction de la décorine avec le collagène.

Un des rôles du tissu conjonctif dermique est de protéger l'organisme contre les agressions externes en formant en même temps une interface informative.

Pour ce faire, le derme possède une forte résistance mécanique en gardant, toutefois, une grande souplesse.

Sa résistance est assurée par le réseau dense des fibres de collagène, mais ce sont les PGs et l'acide hyaluronique, en assurant l'hydratation, la distribution et la souplesse des fibres qui font la différence entre la peau et, par exemple, le cuir.

Les PGs constituent 0,5 à 2 % du poids sec du derme, le collagène en représentant à lui seul jusqu'à 80 %.

La concentration et la distribution dans la peau humaine des GAGs et des PGs varient avec l'âge.

L'acide hyaluronique ou hyaluronanne (HA) est le principal GAG du derme, ce dernier renfermant la moitié d'HA de l'organisme.

La synthèse d'HA est effectuée notamment par les fibroblastes, à proximité de la face interne de la membrane plasmique. Elle s'effectue de façon continue. Ce polysaccharide gigantesque (plusieurs millions de daltons) possède une viscosité intrinsèque très élevée, assurant l'hydratation et l'assemblage des différents éléments du tissu conjonctif par formation de complexes supramoléculaires.

Le dermatanne sulfate (DS), initialement isolé du derme, est aussi très abondant dans la peau. II constitue 40 à 50 % des GAGs dermiques.

Parallèlement aux mécanismes contribuant à l'élaboration de ces matrices extracellulaires spécialisées, il existe des processus de remodelage continuel dont la régulation dépend de la balance entre synthèse et dégradation des éléments protéiques de la matrice.

Plusieurs familles de protéases matricielles sont maintenant décrites ainsi que les facteurs intervenant dans leur activation-inactivation.

Au cours du vieillissement chronologique et/ou actinique, le derme et l'épiderme subissent de nombreuses modifications et dégradations qui se traduisent, avec l'age, par une flaccidité et une perte de souplesse cutanée.

Parmi les éléments dégradés (notamment collagène et élastine), les PGs et les GAGs sont également altérés. En effet, au cours du vieillissement, les fibroblastes et les kératinocytes produisent de moins en moins de PGs et de GAGs et leur synthèse est imparfaite. Il en résulte une désorganisation importante : le dépôt des GAGs sur le squelette protéique formant le PG est anormal, ce qui a pour conséquence, une moins grande avidité pour l'eau de ces PGs et donc une diminution de l'hydratation et de la tonicité des tissus.

Restaurer une production normale de PGs et de GAGs par les fibroblastes et les kératinocytes contribue, en partie, à compenser la perte en hydratation cutanée.

La dégradation de ces matrices contribue donc au phénomène d'assèchement et de perte de souplesse de la peau.

On comprend donc l'importance de pouvoir disposer de produits dont les effets visent à maintenir le taux de PGs et de GAGs dans la peau et lui maintenir ainsi entre autre une bonne hydratation et une bonne souplesse.

A cet égard la demanderesse a découvert, de manière surprenante et inattendue, que des dérivés C-glycosides sont capables d'augmenter la synthèse des glycosaminoglycannes contenant un résidu D-glucosamine et/ou N-acétyl-D-glucosamine, avantageusement de l'acide hyaluronique, et/ou des protéoglycannes, avantageusement des protéoglycannes contenant de l'acide hyaluronique, par les fibroblastes et/ou les kératinocytes.

Dans l'art antérieur, le document WO99/24009 de LVMH décrit l'utilisation du D-xylose, de ses esters et des oligosaccharides contenant du D-xylose pour améliorer la fonctionnalité des cellules de l'épiderme et plus particulièrement comme agent cosmétique ou dermatologique destiné à stimuler la synthèse et/ou la sécrétion des PGs et/ou des GAGs par les kératinocytes, notamment les kératinocytes de l'épiderme, ledit agent étant incorporé dans une composition cosmétique ou pharmaceutique.

En outre, les documents US 4,446,312 et US 4,454,123 décrivent des dérivés de type C-*β*-D-xylopyranoside de formule suivante : 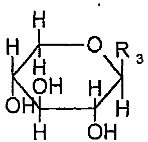 dans laquelle R₃ représente un groupe alkyle ayant respectivement de 1 à 5 et de 6 à 25 atomes de carbone.

Les deux documents US 4,446,312 et US 4,454,123 décrivent que les composés C-*β*-D-xylopyranoside précités induisent la biosynthèse de la chondroïtine sulfate tout en réduisant la quantité de protéoglycanne présents à la surface des membranes cellulaires constitutives des tissus.

Cependant, à la connaissance de la Demanderesse, il n'a jamais été décrit dans l'état de la technique l'utilisation de dérivés C-glycosides pour stimuler la synthèse des glycosaminoglycannes contenant un résidu D-glucosamine et/ou N-acétyl-D-glucosamine, avantageusement de l'acide hyaluronique, et/ou des protéoglycannes, avantageusement des protéoglycannes contenant de l'acide hyaluronique, par les fibroblastes et/ou les kératinocytes.

L'invention a donc pour premier objet l'utilisation d'au moins un dérivé C-glycoside répondant à la formule (I) suivante : 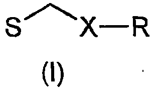 dans laquelle,
- S représente le D-xylose
- la liaison S-CH₂X représente une liaison de nature C-anomèrique,
- X représente un groupement choisi parmi: -CO-, -CH(NR₁R₂)-, -CHR'-, -C(=CHR')-, -CH(OH)-
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle ou benzyle, la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués,
- R', R₁, R₂, identiques ou différents ont la même définition que celle donnée pour R, et peuvent également représenter un hydrogène et un radical hydroxyle,
- R'₁, R'₂, R"₁, R"₂, R"'₁, R"₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 30 atomes de carbone,
dans une composition cosmétique comprenant un milieu physiologiquement acceptable, ladite composition étant destinée à stimuler la synthèse des glycosaminoglycannes contenant un résidu D-glucosamine et/ou N-acétyl-D-glucosamine, avantageusement de l'acide hyaluronique, et/ou des protéoglycannes, avantageusement des protéoglycannes contenant de l'acide hyaluronique, par les fibroblastes et/ou les kératinocytes. Un autre objet de l'invention se rapporte à l'utilisation d'au moins un dérivé C-glycoside de formule (1) telle que définie précédemment dans une composition cosmétique comprenant un milieu physiologiquement acceptable, en tant qu'agent destiné à stimuler la synthèse des glycosaminoglycannes contenant un résidu D-glucosamine et/ou N-acétyl-D-glucosamine, avantageusement de l'acide hyaluronique, et/ou des protéoglycannes, avantageusement des protéoglycannes contenant de l'acide hyaluronique; par les fibroblastes et/ou les kératinocytes.

Un autre objet de l'invention se rapporte à l'utilisation d'un dérivé C-glycoside de formule (I) telle que définie précédemment, pour la fabrication d'une composition pharmaceutique ou dermatologique comprenant un milieu physiologiquement acceptable, la dite composition étant destinée à stimuler la synthèse des glycosaminoglycannes contenant un résidu D-glucosamine et/ou N-acétyl-D-glucosamine, avantageusement de l'acide hyaluronique, et/ou des protéoglycannes, avantageusement des protéoglycannes, contenant de l'acide hyaluronique, par les fibroblastes et/ou les kératinocytes

L'invention concerne également les isomères optiques et/ou géométriques des dérivés C-glycosides répondant à la formule (I), seuls ou en mélange en toutes proportions, ainsi que les sels physiologiquement acceptables de ces dérivés. Par milieu physiologiquement acceptable, on comprend un milieu compatible avec la peau, les muqueuses, les ongles, le cuir chevelu et/ou les cheveux.

La liaison C-anomèrique peut être α ou β.

Bien entendu, selon l'invention, les dérivés C-glycosides répondant à la formule (I) peuvent être utilisés seuls ou en mélange et en toute proportion.

Selon l'invention, les dérivés C-glycosides répondant à la formule (I) peuvent être d'origine naturelle ou synthétique, totalement ou partiellement purifiés ou toute préparation les contenant.

Par origine naturelle, on entend un dérivé extrait de matériel naturel dans lequel il se trouve présent, par exemple des plantes. Par origine synthétique on entend un dérivé préparé par synthèse chimique ou par biotechnologie.

L'expression " totalement ou partiellement purifiés " signifie ici que, durant sa synthèse ou par rapport à son état naturel (plante ou cellules fraîches ou desséchées), le dérivé C-glycoside répondant à la formule (I) dans la composition de l'invention, a été concentré et/ou a été débarrassé, respectivement d'au moins une partie des produits réactionnels secondaires issus de sa synthèse ou d'au moins une partie des autres constituants du matériel naturel dans lequel il se trouve présent.

Selon une forme préférée de l'invention, on utilise des dérivés C-glycosides répondant à la formule (I) pour lesquels R', R₁, R₂, identiques ou différents ont la même définition que celle donnée pour R, et peuvent également représenter un radical hydroxyle.

Selon une autre forme préférée de l'invention, on utilise des dérivés C-glycosides répondant à la formule (I) pour lesquels X représente un groupement choisi parmi -CO-, -CH(OH)-, -CH(NR₁R₂)-, -CH₂-, -C(=CHR')- et représente très avantageusement un groupement -CO-, -CH(OH)- -CH(NH₂)-" S et R conservant par ailleurs l'ensemble des définitions précédemment données.

Selon encore une autre forme préférée de l'invention, on utilise des dérivés C-glycosides répondant à la formule (I) pour lesquels R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 6 atomes de carbone, un radical phényle ou benzyle la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués, S et X conservant par ailleurs l'ensemble des définitions précédemment données.

Dans une autre forme préférée de l'invention, on utilise des dérivés C-glycosides répondant à la formule (I) pour lesquels R', R₁, R₂, identiques ou différents ont la même définition que celle donnée pour R, et peuvent également représenter un hydrogène et un radical hydroxyle, S, X et R conservant par ailleurs l'ensemble des définitions précédemment données.

Dans ces formes préférées de l'invention, on utilise des dérivés C-glycosides répondant à la formule (I) pour lesquels R', R₁, R₂, R'₁, R'₂, R"₁. R"₂, R"'₁, R"'₂ étant définis comme précédemment, S, X et R conservant par ailleurs l'ensemble des définitions précédemment données.

Par "ensemble des définitions précédemment données" pour S, X et R on entend ici aussi bien les définitions générales que les définitions préférées.

Parmi les dérivés C-glycosides de formule (I), utilisés selon l'invention, on préfère tout particulièrement :
- le C-β-D-xylopyranoside-n-propane-2-one ;
- le C-α-D-xylopyranoside-n-propane-2-one ;
- 1-phenyl-2-(C-β-D-xylopyranoside)-ethane-1-one ;
- 1-phenyl-2-(C-α-D-xylopyranoside)-ethane-1-one ;
- 1-[2-(3-hydroxy-propylamino)-propyl]-C-β-D-xylopyranose ;
- 1-[2-(3-hydroxy-propylamino)-propyl]-C-α-D-xylopyranose ;
- le C-β-D-xylopyranoside-2-hydroxy-propane ;
- le C-α-D-xylopyranoside-2-hydroxy-propane ;
- le C-β-D-xylopyranoside-2-amino-propane ;
- le C-α-D-xylopyranoside-2-amino-propane ;
- le C-β-D-xylopyranoside-2-phénylamino-propane ;
- le C-α-D-xylopyranoside-2- phénylamino-propane ;
- l'ester éthylique de l'acide 3-méthyl-4-(C-β-D-xylopyranoside)-butyrique ;
- l'ester éthylique de l'acide 3-méthyl-4-(C-α-D-xylopyranoside)-butyrique
- l'acide 6-(C-β-D-xylopyranoside)-5-céto-hexanoique ;
- l'acide 6-(C-a-D-xylopyranoside)-5-céto-hexanoique ;
- l'acide 6-(C-β-D-xylopyranoside)-5-hydroxy-hexanoique ;
- l'acide 6-(C-α-D-xylopyranoside)-5-hydroxy-hexanoique ;
- l'acide 6-(C-β-D-xylopyranoside)-5-amino-hexanoique ;
- l'acide 6-(C-α-D-xylopyranoside)-5-amino-hexanoique ;
- l'acide 6-(C-β-D-xylopyranoside)-5-phenylamino-hexanoique ;
- l'acide 6-(C-α-D-xylopyranoside)-5-phenylamino-hexanoique ;
- le 1-(C-β-D-xylopyranoside)-hexane-2,6-diol ;
- le 1-(C-α-D-xylopyranoside)-hexane-2,6-diol ;
- l'acide 5-(C-β-D-xylopyranoside)-4-céto-pentanoique ;
- l'acide 5-(C-α-D-xylopyranoside)-4-céto-pentanoique ;
- l'acide 5-(C-β-D-xylopyranoside)-4-hydroxy-pentanoique ;
- l'acide 5-(C-α-D-xylopyranoside)-4-hydroxy-pentanoique ;
- l'acide 5-(C-β-D-xylopyranoside)-4-amino-pentanoique ;
- l'acide 5-(C-α-D-xylopyranoside)-4-amino-pentanoique ;
- l'acide 5-(C-β-D-xylopyranoside)-4-phénylamino-pentanoique ;
- l'acide 5-(C-α-D-xylopyranoside)-4-phénylamino-pentanoique ;
- le 1-(C-β-D-xylopyranoside)-pentane-2,5-diol ;
- le 1-(C-α-D-xylopyranoside)-pentane-2,5-diol;

Ainsi, les dérivés C-glycoside répondant à la formule (I) présentent des activités remarquables pour stimuler la synthèse des GAGs contenant un résidu D-glucosamine et/ou N-acétyl-D-glucosamine, avantageusement de l'acide hyaluronique, et/ou des PGs, avantageusement des PGs contenant de l'acide hyaluronique, par les fibroblastes et/ou les kératinocytes.

Plus précisément, il est apparu que les dérivés C-glycosides de formule (I), du fait de leur effet de stimulation de la synthèse des GAGs' contenant un résidu D-glucosamine et/ou N-acétyl-D-glucosamine, avantageusement de l'acide hyaluronique, et/ou des PGs, avantageusement des PGs contenant de l'acide hyaluronique, par les fibroblastes et/ou les kératinocytes permettaient :
- de lutter contre le vieillissement de l'épiderme. Il est en effet connu que le vieillissement de l'épiderme est, pour une part importante, lié à une perte d'acide hyaluronique,
- de maintenir et/ou stimuler l'hydratation et/ou de lutter contre le dessèchement de la peau lié à une insuffisance de l'action des GAGs, en particulier de l'acide hyaluronique. Un tel dessèchement est en particulier observé sur les peaux âgées et est lié essentiellement à une perte d'acide hyaluronique,
- d'améliorer la tonicité de la peau. Il a été en effet observé que l'augmentation de la synthèse des PGs et des GAGs permettait de créer un environnement cellulaire hydraté favorable aux échanges de nutriments, d'ions, de cytokine et de facteurs de croissance sécrétés par des cellules épidermiques. Un tel environnement est favorable aussi à l'élimination des métabolites toxiques. Cet effet se traduit donc par une peau tonique et en bonne santé,
- de maintenir ou restaurer la souplesse et l'élasticité de la peau. Cet effet est lié à la stimulation de la synthèse des PGs et des GAGs qui permet de créer un environnement hydraté pour les constituants matriciels, en particulier au niveau de la jonction dermo-épidermique pour favoriser les micro-déplacements entre les éléments de cette matrice lors d'un stress mécanique. Un tel effet contribue donc à faire une peau plus souple et plus élastique,
- d'améliorer la minéralisation de l'épiderme, rendant ainsi la peau plus saine et améliorant sa vitalité. Cet effet est lié à l'amélioration de la synthèse des GAGs qui permet d'assurer une bonne minéralisation de l'épiderme. En effet, les GAGs, par leurs groupements charges, peuvent fixer les ions et contribuer à l'osmolarité de l'épiderme. Là encore, une bonne minéralisation de la peau est synonyme de peau saine présentant une bonne vitalité,
- de faciliter les échanges intercellulaires. Cet effet est à rapprocher également de la stimulation de la synthèse des GAGs qui permet d'assurer une différenciation correcte de l'épiderme puisqu'une destruction de l'acide hyaluronique provoque une ouverture des espaces intercellulaires et une acantose épidermique.
   Cet effet permet d'obtenir une peau plus tonique, plus dense et plus compacte,
- d'améliorer la structure tridimensionnelle de la jonction dermo-épidermique. Ceci est également à relier à l'amélioration de la synthèse des PGs et des GAGs qui permet d'assurer l'organisation spatiale des constituants matriciels en assurant, par exemple, au niveau de la jonction dermo-épidermique, le renforcement de la liaison entre la laminine-6 et la nidogène (la nidogène est une glycoprotéine qui avec la laminine fixe les cellules endothéliales au collagène de type IV).
- de faciliter la cicatrisation sans formation de cicatrices, permettant ainsi la réparation de micro-traumatismes épidermiques qui apparaissent lorsqu'il y a rupture de la continuité cutanée. Un tel effet permet de lutter contre les gerçures et l'aspect craquelé de la peau,
- de faciliter la migration des kératinocytes, permettant la formation d'une couche cornée de bonne qualité,
- de moduler l'action des facteurs de croissance et des cytokines produits par les cellules de la peau. Un tel effet permet aux cellules de disposer des signaux dont elles ont besoin pour assurer leur fonction.

L'invention a donc pour objet, l'utilisation, dans une composition cosmétique comprenant un milieu physiologiquement acceptable, d'au moins un dérivé C-glycoside répondant à la formule (I) telle que définie précédemment, le dérivé ou la composition étant destinés à contribuer à au moins une des activités suivantes:
- lutter contre le vieillissement de l'épiderme,
- maintenir et/ou stimuler l'hydratation et/ou lutter contre le dessèchement de la peau lié à une insuffisance de l'action des GAGs contenant un résidu D-glucosamine et/ou N-acétyl-D-glucosamine, en particulier de l'acide hyaluronique,
- améliorer la tonicité de la peau,
- de maintenir ou restaurer la souplesse et l'élasticité de la peau,
- améliorer la minéralisation de l'épiderme, rendant ainsi la peau plus saine et améliorant sa vitalité,
- faciliter les échanges intercellulaires,
- lutter contre les gerçures et l'aspect craquelé de la peau.

L'invention a également pour objet l'utilisation d'au moins un dérivé C-glycoside répondant à la formule (I) telle que définie précédemment pour la fabrication d'une composition pharmaceutique notamment dermatologique comprenant un milieu physiologiquement acceptable, la dite composition étant destinée à traiter les insuffisances de la synthèse des GAGs contenant un résidu D-glucosamine et/ou N-acétyl-D-glucosamine, avantageusement de l'acide hyaluronique, et/ou des PGs, avantageusement des PGs contenant de l'acide hyaluronique, par les fibroblastes et/ou les kératinocytes, en vue de corriger les effets négatifs desdites insuffisances, en particulier d'améliorer l'état fonctionnel des cellules de la peau, notamment de l'épiderme.

Cette composition pharmaceutique pourra en particulier être destinée à faciliter la cicatrisation et à réparer les micro-traumatismes épidermiques ou à traiter les ulcérations cutanées, en particulier des ulcérations des jambes, ou à réduire les stria distensae de la grossesse.

La quantité de dérivés C-glycosides de formule (I) utilisable selon l'invention est bien entendu fonction de l'effet recherché et peut donc varier dans une large mesure.

Pour donner un ordre de grandeur, au moins un dérivé C-glycoside de formule (I) peut être utilisé selon l'invention en une quantité représentant de 0,00001% à 25% du poids total de la composition et préférentiellement en une quantité représentant de 0,0001% à 10% du poids total de la composition.

Pour maintenir et/ou stimuler l'hydratation et/ou la souplesse et l'élasticité de la peau et/ou des muqueuses, lutter contre le vieillissement de l'épidérme, maintenir et/ou stimuler l'hydratation et/ou lutter contre le dessèchement, améliorer la tonicité de la peau, maintenir ou restaurer la souplesse et l'élasticité de la peau, améliorer la minéralisation de l'épiderme, rendant ainsi la peau plus saine et améliorant sa vitalité, faciliter les échanges intercellulaires, améliorer la structure tridimensionnelle de la jonction dermo-épidermique, faciliter la cicatrisation sans formation de cicatrices, faciliter la migration des kératinocytes, permettant la formation d'une couche cornée de bonne qualité, moduler l'action des facteurs de croissance et/ou des cytokines produits par les cellules de la peau, par la stimulation de synthèse des GAGs contenant un résidu D-glucosamine et/ou N-acétyl-D-glucosamine, avantageusement de l'acide hyaluronique, et/ou des PGs, avantageusement des PGs contenant de l'acide hyaluronique, la composition cosmétique selon l'invention est à appliquer sur la peau d'un individu et est éventuellement laissé en contact plusieurs heures et est éventuellement rincé.

Ainsi, la présente invention a pour autre objet un procédé de traitement cosmétique de la peau et/ou du cuir chevelu et/ou des muqueuses, destiné à :
- lutter contre le vieillissement de l'épiderme,
- maintenir et/ou stimuler l'hydratation et/ou lutter contre le dessèchement de la peau lié à une insuffisance de l'action des GAGs contenant un résidu D-glucosamine et/ou N-acétyl-D-glucosamine, en particulier de l'acide hyaluronique,
- améliorer la tonicité de la peau,
- de maintenir ou restaurer la souplesse et l'élasticité de la peau,
- améliorer la minéralisation de l'épiderme, rendant ainsi la peau plus saine et améliorant sa vitalité,
- faciliter les échanges intercellulaires,
- lutter contre les gerçures et l'aspect craquelé de la peau,
caractérisé en ce que l'on applique sur la peau et/ou le cuir chevelu et/ou les muqueuses une composition cosmétique comprenant au moins un dérivé C-glycoside répondant à la formule (I), à laisser celle-ci en contact avec la peau et/ou les muqueuses et/ou le cuir chevelu, et éventuellement à rincer.

Le procédé de traitement présente les caractéristiques d'un procédé cosmétique dans la mesure où il permet d'améliorer l'esthétique ou le confort de la peau et/ou des muqueuses et/ou du cuir chevelu.

Au cours de ces vingt dernières années, la chimie des dérivés C-glycosides a connu un essor considérable.

Deux raisons principales expliquent cet intérêt à savoir;
- contrairement aux dérivés O-glycosides, les dérivés C-glycosides sont parfaitement stables dans les milieux biologiques car non métabolisables ;
- Il a par ailleurs été montré que les structures C-glycosidiques possèdent des propriétés conformationnelles très proches de leurs analogues O-glycosidiques (voir en particulier l'ouvrage "C-Glycoside Synthesis", Postema M. H. D., CRC Press. 1995).

Les dérivés C-glycosides sont bien connus dans la littérature scientifique; on peut notamment citer les publications suivantes :
- Allevi et al. (J. Chem. Soc., Chem. Commun., 1987, pp. 101-102) qui décrit des dérivés de C-glucopyranosyles;
- Nakamura et al. (Tetrahedron Letfers, 1996, 37, pp. 3153-3156);
- Tsang et al. (J. Org. Chem. 1985, 50, pp. 4659-4661) décrit des dérivés pyranose bicycliques;
- Lay et al. (J. Chem. Soc., Perkin Trans. I., 1994, pp. 333-338) qui décrit des dérivés de disaccharides;
- Karagiri et al. (J. Chem. Soc., Perkin Trans. I., 1984, pp. 553-560) qui décrit des pyranozofurines et ses dérivés; et
- Clingerman et al. (J. Org. Chem. 1983, 48, pp. 3141-3146) qui décrit des dérivés de ribofuranosyle.

La plupart des dérivés C-glycosides décrits dans l'art antérieur présentent des fonctions hydroxyles toujours protégées. Ces produits connus sont préparés par des méthodes de synthèse particulièrement laborieuses puisqu'elles nécessitent dans tous les cas de protéger les fonctions hydroxyles.

A la connaissance de la demanderesse, très peu de documents décrivent des dérivés C-glycoside présentant des fonctions hydroxyles non protégés.

On peut citer les documents suivants.

Le document J. Chem. Soc., Perkin Trans. I, 1994, pages 2647-2655 décrit un procédé de synthèse conduisant notamment aux deux dérivés C-glycosides suivants :
- 1-(2-Acétamido-2-déoxy-α-D-galactopyranosyl)-octane et,
- 1-(2-Acétamido-2-déoxy-α-D-glucopyranosyl)-octane.

Le document Liebigs Ann. Chem. 1985, pages 2403-2419 décrit un procédé de synthèse au cours duquel le 1-(α-D-Glucopyranosyl)-2-phényl-éthane est formé transitoirement.

Le document J.Org.Chem. 1991, 56, pages 6412-6422 décrit un procédé de synthèse conduisant notamment aux dérivés C-glycosides suivants :
- le 1-(α-C-D-Glucopyranosyl)-propane ;
- le 1-(β-C-D-Glucopyranosyl)-propane ;
- le 1-(α-C-D-Glucopyranosyl)-2,3-propanediol ;
- le 1-(β-C-D-Glucopyranosyl)-2,3-propanediol ;
- le 1-(2-Déoxy-α-C-D-Glucopyranosyl)-2,3-propanediol ;
- le 1-(2-Déoxy-β-C-D-Glucopyranosyl)-2,3-propanediol.

Le document J.Org.Chem. 1991, 56, pages 6422-6434 décrit des études conformationnelles par RMN et un procédé de synthèse conduisant notamment aux deux dérivés C-disaccharides 1,6-lié suivants :

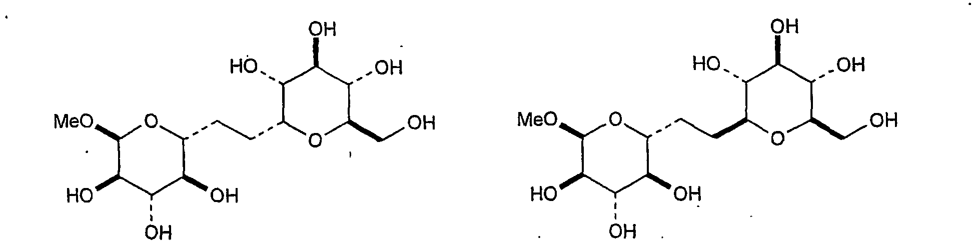

Le document Carbohydrate Research, 200 (1990) pages 11-126 décrit un procédé de synthèse conduisant notamment au 1-(C-β-D-galactopyranosyl)-propane.

Le document Helvetica Chimica Acta, vol.65, Fasc.7 (1982), Nr.229, pages 2333-2237 décrit un procédé de synthèse conduisant notamment au 1-(C-β-D-ribopyranosyl)-n-propanone.

Comme mentionnés précédemment, les documents US 4,446,312 et US 4,454,123 décrivent également des dérivés de type C-β-O-xylopyranoside.

Les dérivés C-glycosides décrits dans un document publié sur Internet le 2 Octobre 2000 (http://www.rsc.org/is/journals/current/chemcomm/cccon.htm) sont :
- la 3'-(β-D-Glucopyranosyl)-propane-2'-one,
- la 3'-(β-D-Mannopyranosyl)-propane-2'-one, et
- la 3'-(D-Glucopyranosyl-(1→4)-β-D-Glucopyranosyl)-propane-2'-one.

Ces trois dérivés C-glycosides sont obtenus de manière quantitative en milieu aqueux alcalin par condensation de la pentane-2,4-dione avec respectivement le D-Glucose, le D-Mannose ou le D-Cellobiose.

La Demanderesse a maintenant synthétisé de nouveaux dérivés C-glycosides répondant à la formule (II), capables de stimuler la synthèse des GAGs contenant un résidu D-glucosamine et/ou N-acétyl-D-glucosamine, avantageusement de l'acide hyaluronique, et/ou des PGs, avantageusement des PGs contenant de l'acide hyaluronique, par les fibroblastes et/ou les kératinocytes et facilement accessibles d'un point de vue synthétique par la mise en oeuvre notamment d'un procédé décrit dans le document précité publié sur internet ou d'un procédé modifié de cette référence.

Ainsi, un autre objet de l'invention se rapporte à de nouveaux dérivés C-glycosides répondant à la formule (II) suivante :

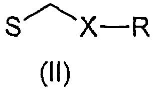

S, représente un groupement choisi parmi -CH(OH)-, -CH(NR₁R₂)- et R, ayant les mêmes définitions que celles données précédemment pour les dérivés C-glycosides de formule (I),

L'invention concerne également les isomères optiques et géométriques, seuls ou en mélange, en toutes proportions, les sels physiologiquement acceptables de ces dérivés.

Selon encore une autre forme préférée de l'invention, on utilise des dérivés C-glycosides répondant à la formule (II) pour lesquels R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 6 atomes de carbone,
la dite chaîne pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués, S et X conservant par ailleurs l'ensemble des définitions précédemment données ;
ledit cycle pouvant être éventuellement interrompu par un ou plusieurs hétéroatomes choisi parmi l'oxygène, le souffre, l'azote, le silicium, et éventuellement substituée par au moins un radical choisi parmi -SR"₁, -NR"'₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués, S et X conservant par ailleurs l'ensemble des définitions précédemment données ; ledit cycle pouvant encore être éventuellement interrompu par un ou plusieurs hétéroatomes choisi parmi le souffre, l'azote, le silicium, et éventuellement substituée par au moins un radical choisi parmi -OR'₁, -SR"₁, -NR"'₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués, S et X conservant par ailleurs l'ensemble des définitions précédemment données.

Dans une autre forme préférée de l'invention, on utilise des dérivés C-glycosides répondant à la formule (II) pour lesquels R₁, R₂, identiques ou différents ont la même définition que celle donnée pour R, et peuvent également représenter un hydrogène et un radical hydroxyle, S, X et R conservant par ailleurs l'ensemble des définitions précédemment données.

Avantageusement, on utilise des dérivés C-glycosides répondant à la formule (II) pour lesquels R₁, R₂, identiques ou différents ont la même définition que celle donnée pour R, et peuvent également représenter et un radical hydroxyle, S, X et R conservant par ailleurs l'ensemble des définitions précédemment données. Dans ces formes préférées de l'invention, on utilise des dérivés C-glycosides répondant à la formule (II) pour lesquels, R₁, R₂, R'₁. R'₂, R"₁, R"₂, R"'₁, R"'₂ étant définis comme précédemment, S, X et R conservant par ailleurs l'ensemble des définitions précédemment données.

Parmi les dérivés C-glycosides de formule (II) selon l'invention, on préfère tout particulièrement :
- 1-[2-(3-hydroxy-propylamino)-propyl]-C-β-D-xylopyranose ;
- 1-[2-(3-hydroxy-propylamino)-propyl]-C-α-D-xylopyranose ;
- le C-β-D-xylopyranoside-2-hydroxy-propane ;
- le C-α-D-xylopyranoside-2-hydroxy-propane ;
- le C-β-D-xylopyranoside-2-amino-propane ;
- le C-α-D-xylopyranoside-2-amino-propane ;
- le C-β-D-xylopyranoside-2-phénylamino-propane ;
- le C-α-D-xylopyranoside-2-phénylamino-propane ;
- l'acide 6-(C-β-D-xylopyranoside)-5-hydroxy-hexanoique ;
- l'acide 6-(C-α-D-xylopyranoside)-5-hydroxy-hexanoique ;
- l'acide 6-(C-β-D-xylopyranoside)-5-amino-hexanoique ;
- l'acide 6-(C-α-D-xylopyranoside)-5-amino-hexanoique ;
- l'acide 6-(C-β-D-xylopyranoside)-5-phenylamino-hexanoique ;
- l'acide 6-(C-α-D-xylopyranoside)-5-phenylamino-hexanoique ;
- l'acide 5-(C-β-D-xylopyranoside)-4-hydroxy-pentanoique ;
- l'acide 5-(C-α-D-xylopyranoside)-4-hydroxy-pentanoique ;
- l'acide 5-(C-β-D-xylopyranoside)-4-amino-pentanoique ;
- l'acide 5-(C-α-D-xylopyranoside)-4-amino-pentanoique ;
- l'acide 5-(C-β-D-xylopyranoside)-4-phénylamino-pentanoique ;
- l'acide 5-(C-α-D-xylopyranoside)-4-phénylamino-pentanoique ;

Les dérivés C-glycoside de formule (II) pour lesquels X correspond à -CH(OH)-, -CH(NR₁R₂)-, -CHR'-, -C(=CHR')-, sont obtenus à partir des dérivés C-glycoside de formule (II) pour lesquels X correspond à -CO- par des procédés bien connu de l'homme du métier comme par exemple ceux décrits dans Advanced Organic Chemistry, Reactions, Mechanisms and Structure, Jerry March (IV Edition) 1992. Ces procédés peuvent éventuellement nécessiter la protection et déprotection des fonctions hydroxyles, ces méthodes de protection et déprotection étant également bien connues de l'homme du métier comme par exemple celles décrites dans le document Profective Groups in Organic Synthesis, T. W. Greene, P. G. M. Wuts (Wiley Interscience).

En outre, certaines fonctions hydroxyles des dérivés C-glycoside de formule (II) peuvent être sulfatées après protections sélectives des autres fonctions hydroxyles. Cette réaction de sulfatation après protection est notamment décrite par A. Lubineau dans la référence J. Chem. Soc. Chem. Commun., 1993, page 1419.

Des exemples détaillés de préparation des composés selon l'invention sont donnés par ailleurs dans les exemples.

Un autre objet de l'invention concerne une composition qui comprend au moins un dérivé C-glycoside répondant à la formule (II) tel que défini ci-dessus.

Bien entendu, la composition selon l'invention peut comprendre les dérivés C-glycoside répondant à la formule (II) seuls ou en mélanges en toutes proportions

La quantité de dérivés C-glycosides répondant à la formule (II) peuvent utilisable dans les compositions selon l'invention, dépend bien évidemment de l'effet recherché et doit être en une quantité efficace pour stimuler la synthèse de l'acide hyaluronique et des protéoglycannes par les fibroblastes et les kératinocytes.

Pour donner un ordre de grandeur, la composition de l'invention peut contenir au moins un dérivé C-glycoside répondant à la formule (II) en une quantité représentant de 0,00001 % à 25% du poids total de la composition et préférentiellement en une quantité représentant de 0,0001 % à 10% du poids total de la composition.

La composition selon l'invention peut être destinée à une application cosmétique ou pharmaceutique, particulièrement dermatologique. De préférence la composition selon l'invention est destinée à une application cosmétique.

Avantageusement, les compositions selon l'invention sont des compositions de lavage et/ ou de maquillage et/ ou démaquillage de la peau du corps et/ ou du visage et/ ou des muqueuses (par exemple les lèvres) et/ ou du cuir chevelu et/ou des cheveux et/ ou des ongles, et/ ou des cils et/ ou des sourcils.

Un autre objet de l'invention se rapporte également à l'utilisation de la composition telle que définie précédemment pour le traitement cosmétique des matières kératiniques, telles que les cheveux, la peau du corps et/ ou du visage, les cils, les sourcils, les ongles, les muqueuses.

Un autre objet de l'invention se rapporte à l'utilisation de la composition telle que définie précédemment pour améliorer l'apparence des matières kératiniques.

La composition selon l'invention peut être ingérée, injectée ou appliquée sur la peau (sur toute zone cutanée du corps), les cheveux, les ongles ou les muqueuses (buccale, jugale, gingivale, génitale, conjonctive).

Selon le mode d'administration, la composition de l'invention peut se présenter sous toutes les formes galéniques normalement utilisées, particulièrement en cosmétologie.

Une composition préférée de l'invention est une composition cosmétique destinée à une application topique.

Pour une application topique sur la peau, la composition utilisable selon l'invention peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

La composition selon l'invention comprend bien évidemment un support cosmétiquement acceptable et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules; ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

La composition utilisable selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Les quantités des différents constituants des compositions utilisables selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits après-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosacée, le psoriasis, les lichens, les prurits sévères.

Les compositions utilisables selon l'invention peuvent également consister en des préparations solides constituant des savons ou des pains de nettoyage.

Les compositions utilisables selon l'invention peuvent aussi être conditionnées sous forme d'une composition pour aérosol comprenant également un agent propulseur sous pression.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition utilisable selon l'invention est une solution ou un gel huileux, la phase grasse peut représenter plus de 90% du poids total de la composition.

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs, notamment l'éthanol et l'isopropanol, le propylène glycol.

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Bien entendu, au moins un dérivé C-glycoside de formule (II) peut être utilisé dans la préparation de compositions cosmétiques et/ou pharmaceutiques, particulièrement dermatologiques, destinées à stimuler ou à traiter la synthèse ou de la sécrétion des GAGs et/ou des PGs, en vue de corriger les effets négatifs desdites insuffisances, en particulier d'améliorer l'état fonctionnel des cellules de la peau, notamment du derme.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### Exemple 1 : Exemple de dérivé C-glycoside de xylose

### Synthèse de la C-β-D-xylopyranoside-n-propane-2-one de formule (i)

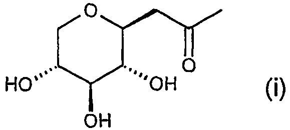

Schéma réactionnel

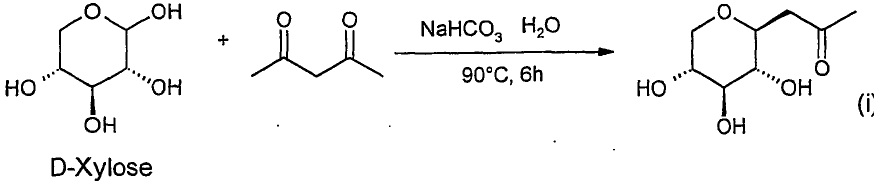

### Mode Opératoire :

Dans un tricol de 25 ml muni d'un réfrigérant, d'un thermomètre ainsi que d'une agitation magnétique, on dissout le D-xylose (1 g, 6,66 mmol) dans 10ml d'eau puis on additionne la penta-2,4-dione (0,82 ml, 8 mmol) et le bicarbonate de sodium (839 mg, 10 mmol). Le mélange est agité pendant 6 heures à 90°C. Le milieu réactionnel est ensuite lavé avec du dichlorométhane (2 fois 5 ml). La phase aqueuse est passée sur une résine Dowex 50X-200 qui a été préalablement conditionnée par des lavages aqueux (la solution finale est à pH 3-4). On concentre puis on co-évapore avec de l'éthanol.

L'huile marron clair obtenue est séchée sous vide (600 mg, soit un rendement de 76%). ,

RMN ¹H : conforme à la structure attendue.

### Exemple 3 : Exemple de protection de dérivé C-glycoside de xylose

### Synthèse de la C-β-D-(3.4.5-triacetoxy)xylopyranoside-n-propane-2-one de formule (iv)

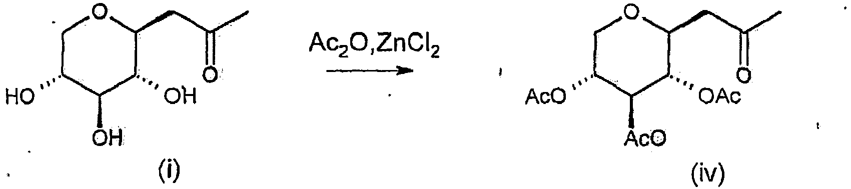

Dans un tricol on solubilise le 1-(3,4,5-trihydroxy-tétrahydro-pyran-2-yl)-propan-2-one (5,56 mmol) dans 10mL d'anhydride acétique et on additionne une quantité catalytique de ZnCl₂. On laisse agiter pendant une nuit à température ambiante. Après une nuit d'agitation on dilue avec l'acétate d'éthyle, la phase organique est lavée avec une solution saturée de NaHCO₃, puis lavé trois fois avec de l'eau, séché sur MgSO₄, filtré et évaporé. On obtient le sucre peracetylé avec un rendement quantitatif.

### Exemple 4 : Exemple de réduction de la cétone en hydroxyle de dérivé C-glycoside de xylose (Dérivé C-glycoside de formule (I) pour lesquels X correspond à -CH(OH)-).

### Synthèse de la C-β-D-xylopyranoside-2-hydroxy-propane de formule (vi) :

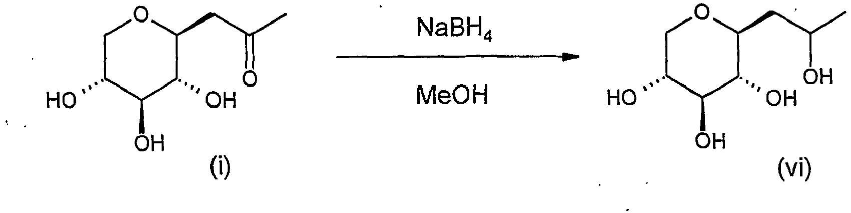

Dans un ballon de 25 ml on dissout le dérivé xylose (5,56 mmol) dans 10mL de méthanol et on refroidit à 0°C. On additionne par petites portions le borohydrure de sodium (7 mmol) puis on laisse le milieu réactionnel revenir à température ambiante et sous agitation pendant 15 heures. On refroidit le mélange à 0°C auquel on ajoute 5mL d'eau et une solution d'acide chlorhydrique (1 N) jusqu'à obtenir un milieu réactionnel à pH 1 puis on extrait trois fois au butanol. La phase organique est lavée deux fois avec une solution de chlorure de sodium saturée puis séchée sur sulfate de sodium, filtrée et concentrée au maximun. Obtention d'une huile jaune.

### Exemple 5 : Exemple d'alkylation réductrice de la cétone en amine de dérivé C-glycoside de xylose (Dérivé C-glycoside de formule (I) pour lesquels X correspond à -CH(NR₁R₂)-).

### Synthèse de la C-β-D-xylopyranoside-2-phenylamino-propane de formule (vii)

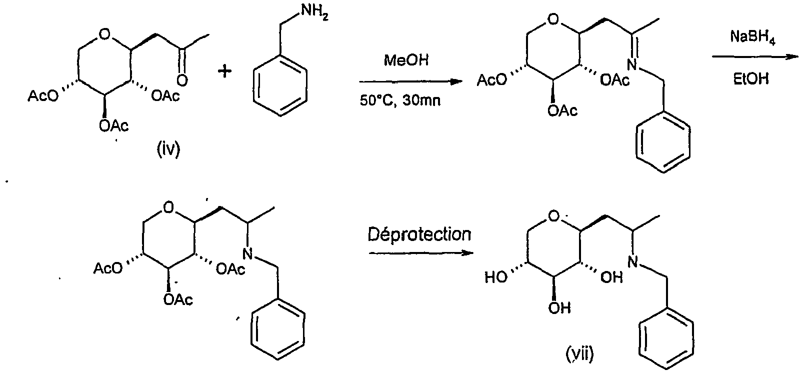

Dans un tricol de 25 mL muni d'un réfrigérant, d'un thermomètre ainsi que d'une agitation magnétique, on dissout le C-β-D-(3.4.5-triacetoxy)xylopyranoside-n-propane-2-one (1 g, 3,20 mmol) dans 15mL de méthanol puis on additionne la benzylamine (0,34g, 3,20 mmol). Le mélange est agité pendant 30 minutes à 50°C. On concentre au maximun le milieu réactionnel. Obtention d'une huile qui est séchée sous vide. Cette huile est diluée dans 15mL d'éthanol et on additionne par petites portions le borohydrure de sodium (0,12g, 3,20 mmol). Puis on laisse le milieu réactionnel sous agitation pendant 2 heures. On refroidit le mélange à 0°C auquel on ajoute 5mL d'eau et une solution d'acide chlorhydrique (1 N) jusqu'à obtenir un milieu réactionnel à pH 1 puis on extrait. La phase organique est lavée deux fois avec une solution de chlorure de sodium saturée puis séchée sur sulfate de sodium, filtrée et concentrée au maximun. Obtention d'une huile.

L'étape de déprotection est réalisée suivant le mode opératoire général décrite ci-dessous dans l'exemple 8.

### Exemple 6 : Exemple de transformation de la cétone en chaîne alkyl par la réaction de Wittig de dérivé C-glycoside de xylose (Dérivé C-glycoside de formule (I) pour lesquels X correspond à -CHR'-).

Synthèse de l'ester éthylique de l'acide 3-méthyl-4-(C-β-D-xylopyranoside)-butyrique de formule (viii) :

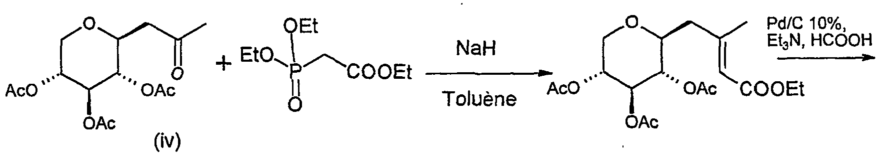

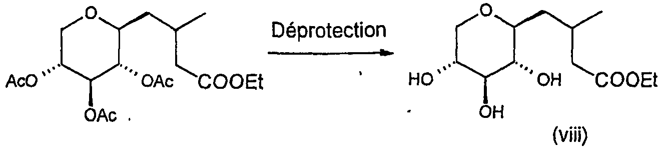

### Réaction de Wittig :

Dans un tricol de 50 mL muni d'un réfrigérant, d'un thermomètre ainsi que d'une agitation magnétique, on dissout 0,03 mmol de NaH (dispersion dans l'huile) dans 50 ml de toluène anhydre, sous azote. On l'addition à cette solution goutte à goutte 0,03 mmol de triethyl phosphonoacetate en gardant la température entre 30-35°C. Après addition, on laisse agité pendant 1 h à température ambiante. A cette solution claire on additionne goutte à goutte 0,03 mmol de 1-(3,4,5-triacétoxy-tetrahydro-pyran-2-yl)-propan-2-one dans 5mL de toluène anhydre en maintenant la température à 20-25°C. Après addition on chauffe à 60-65°C pendant 15 minutes, puis on refroidit à 15-20°C. Le précipité formé est décanté, et le produit pâteux est relavé trois fois avec du toluène à 60°C (attention on décante chaque fois à température ambiante). Les phases de toluène sont rassemblés, évaporés à sec et le produit obtenu est séparé sur colonne de silicagel.

Obtention d'une solide.

### Réduction de la double liaison :

0,1 mmole de produit de départ a été dissous dans 10 ml éthanol et hydrogéné à 8 barr en présence de Pd/C (10%). Après 3 heures la réaction est filtrée sur célite puis le filtrat est évaporé.

L'étape de déprotection est réalisée suivant le mode opératoire général décrite ci-dessous dans l'exemple 8.

### Exemple 7 : Etape de déprotection des fonctions O-acétylés

On prépare une solution de dérivé acétylé de xylose (5 mmol) dans 10 ml du méthanol. On additionne 3 équivalent molaire de méthanolate de sodium (MeONa) et on agite durant 3 à 12h. On évapore le méthanol, on dilue avec de l'eau. La phase aqueuse est passée sur une résine Dowex 50X-200 qui a été préalablement conditionnée par des lavages aqueux (la solution finale est à pH 3-4). On concentre puis on co-évapore avec de l'éthanol.

L'huile obtenue est séchée sous vide.

### Exemple 8 : 1-phenyl-2-(C-β-D-xylopyranoside)-ethane-1-one

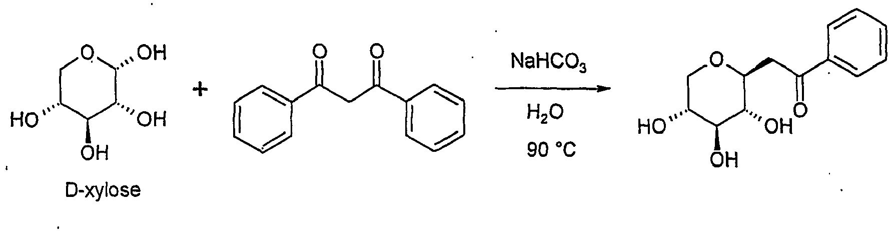

Dans un tricol de 250 ml dissoudre 1 équivalent de D-xylose (0,0333 mol ; 5 g) dans environ 50 ml d'eau ; ajouter 1,5 équivalents d'hydrogénocarbonate de sodium (0,4995 mol ; 4,2 g), 50 ml d'eau et 1,2 équivalents de dibenzoyleméthane (0,0399 mol ; 9 g) par petites spatulées (réaction exothermique).

Chauffer à 90 °C pendant 6 heures.

Laisser le milieu réactionnel revenir à température ambiante, et filtrer le précipité formé (ce précipité correspond au dibenzoylméthane en excès) ; effectuer 2 extractions successives au dichlorométhane et extraire la phase aqueuse avec 2 fractions de 50 ml de butanol ; puis laver la phase butanol avec 2 fractions de 50 ml d'eau saturée en NaCl. Concentrer à sec la phase organique butanol récupérée et solubilisation du précipité formé dans de l'eau et passage sur résine échangeuse d'ions Dowex 50W 2.200 ; concenter à sec les fractions récupérées. Purifier le précipité obtenu sur colonne de silice en utilisant un mélange 3 % méthanol / 97 % dichlorométhane comme éluant.

Obtention de 480 mg d'un précipité beige

### Exemple 9 : 1-[2-(3-hydroxy-propylamino)-propyl]- C-β-D-xylopyranose

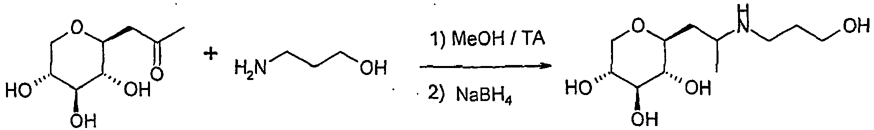

Dans un tricol de 50 ml dissoudre 1 équivalent de 1-(3,4,5-trihydroxy-tetrahydro-pyran-2-yl-propan-2-one (2,6316 mmol ; 500 mg) dans environ 20 ml de méthanol ; ajouter 1 équivalent de 3-amino-1-propanol (2,6316 mmol ; 0,2 ml), et agiter à température ambiante pendant 6 heures. Ajouter 1,4 équivalents de borohydrure de sodium (3,684 mmol ; 140 mg) par petites spatulées (réaction exothermique) et laisser agiter à température ambiante pendant 12 heures. Concentrer à sec le milieu réactionnel et diluer l'huile obtenue dans le minimum d'éthanol ; passer ce mélange sur fritté de silice afin d'éliminer les sels de bore; concentrer à sec le filtrat.

Obtention de 100 mg d'une huile beige (rendement = 15 %).

### Exemple 10 : Etudes de l'effet de dérivés C-glycosides sur la synthèse de l'acide hyaluronique :

L'étude est faite par mesure de l'incorporation de glucosamine radioactive dans la matrice néosynthétisée par des cultures de fibroblastes dermiques humains normaux. L'incorporation de glucosamine radioactive indique une néosynthèse spécifique de glycosaminoglycannes via une incorporation de la forme acétylée de cette glucosamine.

Les cultures de fibroblastes sont effectuées selon les méthodes classiques de culture cellulaire, à savoir en milieu MEM/M199 vendu par la société Gibco, en présence de bicarbonate de sodium (1,87 mg/ml) de L-glutamine (2 mM), de pénicilline (50 Ul/ml) et de 10% de sérum de veau foetal (Gibco).

Le test est effectué sur culture de cellules à 80% de confluence en plaque de 96 puits. Le dérivé C-glycoside aux concentrations de 0,1 à 30 mM est mis en contact avec les cellules pendant 48 heures. Le marquage avec la glucosamine radioactive (D-[6-H³ ]-glucosamine , Amersham TRK398 (814 Gbq/mmol, 22 Ci/mmol)) est effectué pendant 24 heures.

Le taux de glucosamine radioactive incorporée est mesuré en fin de test par adsorption des molécules anioniques sur billes de Q-sepharose suivit d'une désorption des molécules peu et moyennement anioniques avec urée 6M plus NaCl 0,2M. Une fois lavée, le comptage de la radioactivité incorporée dans les molécules très cationiques restées sur le support est effectué.

Les résultats sont évalués par apport à un témoin constitué par des cellules n'ayant pas été traitées avec le dérivé C-glycoside de formule (1).

Un témoin positif (TGFβ à 10 ng/ml) connu pour stimuler la synthèse des GAGs est introduit dans le test comme référence positive.

Les résultats sont présentés dans le tableau suivant :

| Traitement | | valeur | sd | n | % | p |
|---|---|---|---|---|---|---|
| Contrôle (milieu de culture) | | 7793-7681 | 274-176 | 3-3 | 100-100 | - |
| Contrôle positif (TGFβ) | | 27124-34865 | 947-2849 | 3-3 | 348-454 | <0.01 <0.01 |
| D-Xylose | 0.5mM | 4063 | 347 | 3 | 86 | >0.05 |
| | 0.1mM | 6586 | 489 | 3 | 102 | >0.05 |
| C-β-D-xylopyranoside-2-hydroxy-propane | 3 mM | 18271 | 1373 | 3 | 218 | P<0.01 |
| | 1 mM | 14209 | 1799 | 3 | 169 | P<0.01 |
| | 0.3mM | 11634 | 626 | 3 | 139 | P<0.05 |
| 1-Phenyl-2-(C-β-D-xylopyranoside)-ethane-1-one | 1 mM | 7941 | 172 | 3 | 95 | p>0.05 |
| | 0.3mM | 8545 | 653 | 3 | 102 | p>0.05 |
| | 0.1mM | 10113 | 890 | 3 | 120 | p>0.05 |
| 1-[2-(3-hydroxy-propylamino)-propyl]-C-β-D-xylopyranose | 1 mM | 14832 | 343 | 3 | 140 | P<0.01 |
| | 0.3mM | 11585 | 804 | 3 | 109 | p>0.05 |
| | 0.1mM | 11656 | 1084 | 3 | 110 | p>0.05 |
| C-β-D-xylopyranoside-n-propane-2-one | 10mM | 12507-9811 | 1201 | 3 3 | 161 | <0.01 >0.05 |
| | 2mM | 11014 | 639 | 3 | 131 | <0.01 |
| | 0.4mM | 8564 | 625 | 3 | 141 | >0.05 |

Les valeurs mesurées sont données en coups par minute (cpm) sd : déviation standard
p : intervalle de confiance
n : replicates

Ces résultats montrent que :
- la C-β-D-xylopyranoside-n-propane-2-one stimule l'incorporation de glucosamine radioactive entre les concentrations de 0.4, 2 et 10 mM :
- la C-β-D-xylopyranoside-2-hydroxy-propane stimule l'incorporation de glucosamine radioactive aux concentrations de 0.1 ; 0.3 et 1 mM :
- la 1-Phenyl-2-(C-β-D-xylopyranoside)-ethane-1-one et la 1-[2-(3-hydroxy-propylamino)-propyl]- C-β-D-xylopyranose stimulent l'incorporation de glucosamine radioactive aux concentrations de 0.3 ; 1 et 3 mM .

Ainsi, les dérivés C-glycosides selon l'invention stimulent l'incorporation de glucosamine radioactive ce qui indique une néosynthèse de glycosaminoglycannes via une incorporation de la forme acétylée de cette glucosamine.

### Exemple 11 : Compositions selon l'invention

### Composition 1 : Crème Huile dans Eau (H/E) :

| | |
|---|---|
| Monostéarate de glycérol | 6,0% |
| Alcool stéarylique | 4,0% |
| Huile de vaseline | 10,0% |
| Huile de silicone | 5,0% |
| C-β-D-xylopyranoside-n-propane-2-one | 10,0% |
| Glycérine | 8,0% |
| Polymère carboxyvinylique type Carbopol | 0,3% |
| Conservateurs | 0,4% |
| Parfum | 0,5% |
| triéthanolamine | 0,3% |
| Eau qsp | 100% |

### Composition 2 : Crème Eau dans Huile (E/H):

| | |
|---|---|
| Octyl dodécanol | 10,0% |
| Stéarate de magnésium | 4,0% |
| Cire d'abeille naturelle | 5,0% |
| Sesquioléate de sorbitan | 4,5% |
| Mono et distéarate de glycérol et stéarate de potassium | 1,0% |
| Huile de vaseline | 22,0% |
| Huile de Jojoba | 4,0% |
| C-β-D-xylopyranoside-n-propane-2-one | 2,5% |
| Conservateurs | 0,4% |
| Parfum | 0,6% |
| Eau qsp | 100% |

### Composition 3 : Gel Hydratant :

| | |
|---|---|
| C-β-D-xylopyranoside-n-propane-2-one | 5,0% |
| Glycérine | 12,0% |
| Copolymère acrylamide/acrylamido 2-méthyl propane sulfonate de sodium à 40% dans isoparaffine/eau (type SEPIGEL 305) | 5,0% |
| Mélange de poly diméthylsiloxane groupements alpha-omega hydroxylé et de cyclopenta diméthylsiloxane (15/85) | 2,0% |
| Conservateurs | 0,4% |
| Parfum | 0,6% |
| Eau qsp | 100% |

## Revendications

1. Utilisation cosmétique d'un dérivé C-glycoside répondant à la formule (I) suivante: 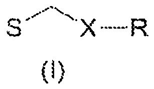 dans laquelle,
- S représente le D-xylose ,
- la liaison S-CH₂X représente une liaison de nature C-anomérique,
- X représente un groupement choisi parmi: -CO-, -CH(OH)-, -CH(NR₁R₂)-, -CHR'-, -C(=CHR')-,
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle ou benzyle, la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR'''₁R'₂, -COOR"₂, -CONHR'"₂, -CN, halogène, perfluoroalkyle, hydrofluoroalkyle et/ou au moins un radical cycloalkyle, aryle, hétérocyclique éventuellement substitués.
- R', R₁, R₂, identiques ou différents ont la même définition que celle donnée pour R, et peuvent également représenter un hydrogène et un radical hydroxyle,
- R'₁, R'₂, R"₁, R"₂, R"'₁, R"'₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 30 atomes de carbone,
ainsi que ses sels physiologiquement acceptables et ses isomères optiques,
ou d'une composition comprenant, dans un milieu physiologiquement acceptable; ledit dérivé C-glycosade de formule (I), pour stimuler la synthèse des glycosaminoglycannes contenant un résidu D-glucosamine et/ou N-acétyl-D-glucosamine, et/ou des protéoglycannes, par les fibroblastes et/ou les kératinocytes.

2. Utilisation selon la revendication précédente, **caractérisée en ce que** pour le dérivé C-glycoside de formule (I) R représente une chaîne alkyle, perfluoroalkyle, hydrofluoroalkyle linéaire ou ramifiée, saturée or insaturée, un cycle cycloalkyle, cycloperfluoroalkyle, cyclohydrofluoroalkyle, comprenant de 1 à 6 atomes de carbone, un radical phényle ou benzyle, la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"'₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués.

3. Utilisation selon la revendication précédente, **caractérisée en ce que** le dérivé C-glycoside de formule (I) est choisi parmi :
- le C-β-D-xylopyranoside-n-propane-2-one ;
- le C-α-D-xylopyranoside-n-propane-2-one ;
- 1-phenyl-2-(C-β-D-xyropyranoside)-ethane-1-one ;
- 1-phenyl-2-(C-α-D-xylopyranoside)-ethane-1-one ;
- 1-[2-(3-hydroxy-propylamino)-propyl]-C-β-D-xylopyranose ;
- 1-[2-(3-hydroxy-propylamino)-propyl]-C-α-D-xylopyranose ;
- le C-β-D-xylopyranoside-2-hydroxy-propane :
- le C-α-D-xylopyranoside-2-hydroxy-propane ;
- le C-β-D-xylopyranoside-2-amino-propane ;
- le C-α-D-xylopyranoside-2-amino-propane ;
- le C-β-D-xylopyranoside-2-phénylamino-propane ;
- le C-α-D-xylopyranoside-2- phénylamino-propane ;
- l'ester éthylique de l'acide 3-méthyl-4-(C-β-D-xylopyranoside)-butyrique ;
- l'ester éthylique de l'acide 3-méthyl-4-(C-α-D-xylopyranoside)-butyrique
- l'acide 6-(C-β-D-xylopyranoside)-5-céto-hexanoique :
- l'acide 6-(C-α-D-xylopyranoside)-5-céto-hexanoique;
- l'acide 6-(C-β-D-xylopyranoside)-5-hydroxy-hexanoique ;
- l'acide 6-(C-α-D-xylopyranoside)-5-hydroxy-hexanoique ;
- l'acide 6-(C-β-D-xylopyranoside)-5-amino-hexanoique ;
- l'acide 6-(C-α-D-xylopyranoside)-5-amino-hexanoique ;
- l'acide 6-(C-β-D-xylopyranoside)-5-phenylamino-hexanoique ;
- l'acide 6-(C-α-D-xylopyranoside)-5-phenylamino-hexanoique ;
- le 1-(C-β-D-xylopyranoside)-hexane-2,6-diol ;
- le 1-(C-α-D-xylopyranoside)-hexane-2,6-diol ;
- l'acide 5-(C-β-D-xylopyranoside)-4-céto-pentanoique ;
- l'acide 5-(C-α-D-xylopyranoside)-4-céto-pentanoique ;
- l'acide 5-(C-β-D-xylopyranoside)-4-hydroxy-pentanoique;
- l'acide 5-(C-α-D-xylopyranoside)-4-hydroxy-pentanoique ;
- l'acide 5-(C-β-D-xylopyranoside)-4-amino-pentanoique ;
- l'acide 5-(C-α-D-xylopyranoside)-4-amino-pentanoique ;
- l'acide 5-(C-β-D-xylopyranoside)-4-phénylamino-pentanoique;
l'acide 5-(C-α-D-xylopyranoside)-4-phénylamino-pentanoique ;
- le 1-(C-β-D-xylopyranoside)-pentane-2,5-diol;
- le 1-(C-α-D-xylopyranoside)-pentane-2,5-diol.

4. Utilisation selon l'une quelconque des revendications précédentes, **caractérisée en ce que** le dérivé C-glycoside de formule (I) est choisi parmi :
- le C-β-D-xylopyranoside-n-propane-2-one ;
- le C-α-D-xylopyranoside-n-propane-2-one ;
- 1-phenyl-2-(C-β-D-xylopyranoside)-ethane-1-one;
- 1-phenyl-2-(C-α-D-xylopyranoside)-ethane-1-one ;
- 1-[2-(3-hydroxy-propylamino)-propyl]- C-β-D-xylopyranose ;
- 1-[2-(3-hydroxy-propylamino)-propyl]- C-α-D-xylopyranose ;
- le C-β-D-xylopyranoside-2-hydroxy-propane;
- le C-α-D-xylopyranoside-2-hydroxy-propane.

5. Utilisation selon l'une quelconque des revendications précédentes, **caractérisée en ce que** le dérivé C-glycoside est le C-β-D-xylopyranoside-2-hydroxy-propane.

6. Utilisation selon l'une quelconque des revendications précédentes, **caractérisée en ce que** le dérivé C-glycoside de formule (I) est présent dans la composition en une teneur allant de 0,00001 % à 25% du poids total de la composition.

7. Utilisation selon l'une quelconque des revendications précédentes, **caractérisée en ce que** le dérivé C-glycoside de formule (I) ou la composition est destiné à stimuler la synthèse de l'acide hyaluronique et/ou des protéoglycannes contenant de l'acide hyaluronique.

8. Procédé de traitement cosmétique de la peau et/ou du cuir chevelu et/ou des muqueuses pour contribuer à au moins une des activités suivantes:
- lutter contre le vieillissement de l'épiderme,
- maintenir et/ou stimuler l'hydratation et/ou lutter contre le dessèchement de la peau lié à une insuffisance de l'action des GAGs contenant un résidu D-glucosamine et/ou N-acétyl-D-glucosamine, en particulier de l'acide hyaluronique,
- améliorer la tonicité de la peau,
- de maintenir ou restaurer la souplesse et l'élasticité de la peau,
- améliorer la minéralisation de l'épiderme, rendant ainsi la peau plus saine et améliorant sa vitalité,
- faciliter les échanges intercellulaires,
- lutter contre les gerçures et l'aspect craquelé de la peau,
**caractérisé en ce que** l'on applique sur la peau et/ou le cuir chevelu et/ou les muqueuses, une composition cosmétique comprenant , dans un milieu physiologiquement acceptable , un dérivé C-glycoside de formule (I) tel que défini selon l'une quelconque des revendications 1 à 5, à laisser celle-ci en contact avec la peau et/ou les muqueuses et/ou le cuir chevelu, et éventuellement à rincer.

9. Procédé selon la revendication précédente, **caractérisé en ce que** le dérivé C-glycoside de formule (I) est présent dans la composition en une teneur allant de 0,00001% à 2.5% du poids total de la composition.

10. Utilisation d'une composition comprenant, dans un milieu physiologiquement acceptable, un dérivé C-glycoside de formule (I) tel que défini selon l'une quelconque des revendications 1 à 5 , pour ie traitement cosmétique des matières kératiniques.

11. Utilisation d'une composition comprenant, dans un milieu physiologiquement acceptable, un dérivé C-glycoside de formule (I) tel que défini selon l'une quelconque des revendications 1 à 5, pour améliorer l'apparence des matières kératiniques.

12. Utilisation selon la revendication précédente, **caractérisée en ce que** matières kératiniques sont choisies parmi les cheveux, les cils, les sourcils, les ongles.

13. Utilisation d'une composition comprenant, dans un milieu physiologiquement acceptable, un dérivé C-glycoside de formule (I) tel que défini selon l'une quelconque des revendications 1 à 5, pour le soin capillaire.

14. Utilisation selon la revendication précédente, **caractérisée en ce que** la composition est une lotion ou un gel antichute.

15. Utilisation selon l'une quelconque des revendications 10 à 14, **caractérisée en ce que** le dérivé C-glycoside de formule (I) est présent dans la composition en une teneur allant de 0,00001% à 25% du poids total de la composition.

16. Dérivé C-glycoside répondant à la formule (II) suivante ; 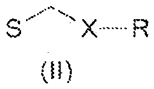 dans laquelle,
- S représente le D-xylose,
- la liaison S-CH₂X représente une liaison de nature C-anomèrique,
- X représente un groupement choisi parmi: -CH(OH)-, -CH(NR₁R₂)-,
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle ou benzyle, la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"'₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués,
- R₁, R₂, identiques ou différents ont la même définition que celle donnée pour R, et peuvent également représenter un hydrogène et un radical hydroxyle,
- R'₁, R'₂, R"₁, R"₂, R"'₁, R"'₂, identiques ou différents, représentent un atome d'hydrogène, un radical choisi parmi un radical alkyle, hydroxyle, perfluoroalkyle et/ou hydrofluoroalkyle, alinéaire ou ramifié, saturé ou insaturé, comprenant de 1 à 30 atomes de carbone,
ainsi que ses sels physiologiquement acceptables et ses isomères optiques.

17. Dérivé C-glycoside selon la revendication précédente, **caractérisée en ce que** R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 6 atomes de carbone, un radical phényle ou benzyle, la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"'₁R'₂, -COOR"₂, -CONHR"'₂, -CN, halogène, perfluorcalkyle, hydrofluoroalkyle et/ou au moins un radical cycloalkyle, aryle, hétérocyclique éventuellement substitués.

18. Dérivé C-glycoside selon l'une quelconque des revendications 16 ou 17, **caractérisé en ce qu'**il est choisi parmi :
- 1-[2-(3-hydroxy-propylamino)~propyl]-C-β-D-xylopyranose ;
- 1-[2-(3-hydroxy-propylamino)-propyl]-C-α-D-xylopyranose ;
- le C-β-D-xylopyranoside-2-hydroxy-propane ;
- le C-α-D-xylopyranoside-2-hydroxy-propane ;
- le C-β-D-xylopyranoside-2-amino-propane ;
- le C-α-D-xylopyranoside-2-amino-propane ;
- le C-β-D-xylopyranoside-2-phénylamino-propane ;
- le C-α-D-xylopyranoside-2-phénylamino-propane ;
- l'acide 6-(C-β-D-xylopyranoside)-5-hydroxy-hexanoique ;
- l'acide 6-(C-α-D-xylopyranoside)-5-hydroxy-hexanoique ;
- l'acide 6-(C-β-D-xylopyranoside)-5-amino-hexanoique ;
- l'acide 6-(C-α-D-xylopyranoside)-5-amino-hexanoique ;
- l'acide 6-(C-β-D-xylopyranoside)-5-phenylamino-hexanoique ;
- l'acide 6-(C-α-D-xylopyranoside)-5-phenylamino-hexanoique ;
- l'acide 5-(C-β-D-xylopyranoside)-4-hydroxy-pentanoique ;
- l'acide 5-(C-α-D-xylopyranoside)-4-hydroxy-pentanoique ;
- l'acide 5-(C-β-D-xylopyranoside)-4-amino-pentanoique ;
- l'acide 5-(C-α-D-xylopyranoside)-4-amino-pentanoique ;
- l'acide 5-(C-β-D-xylopyranoside)-4-phénylamino-pentanoique ;
- l'acide 5-(C-α-D-xylopyranoside)-4-phénylamino-pentanoique.

19. Dérivé C-glycoside selon l'une quelconque des revendications 16 à 18, **caractérisé en ce qu'**il est choisi parmi :
- 1-[2-(3-hydroxy-propylamino)-propyl]- C-β-D-xylopyranose :
- 1-[2-(3-hydroxy-propylamino)-propyl]- C-α-D-xylopyranose ;
- le C-β-D-xylopyranoside-2-hydroxy-propane ;
- le C-α-D-xylopyranoside-2-hydroxy-propane.

20. Dérivé C-glycoside selon l'une quelconque des revendications 16 à 19. **caractérisée en ce qu'**il est le C-β-D-xylopyranoside-2-hydroxy-propane.

21. Composition comprenant, dans un milieu physiologiquement acceptable, un dérivé de C-glycoside selon l'une quelconque des revendications 16 à 20.

22. Composition selon la revendication précédente, **caractérisée en ce que** le dérivé de C-glycoside est présent en une teneur allant de 0,00001% à 25% du poids total de la composition.

23. Composition selon l'un des revendications 21 ou 22, **caractérisée en ce qu'**elle comprend un adjuvant cosmétique choisi parmi les gélifiants hydrophiles ou lipophiles, les actifs hydrophiles ou lipoplaes, les conservateurs, les antioxydants, les solvants, les parfums, ses charges, les filtres, les pigments, les agents chélateurs, les absorbeurs d'odeur et les matières colorantes.

24. Composition selon l'une des revendications 21 à 23, caractérisée qu'elle est sous forme d'émulsion.

## Patentansprüche

1. Kosmetische Verwendung eines C-Glykosidderivats der folgenden Formel (I): 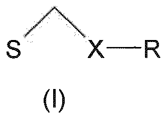 in der
- S D-Xylose bedeutet,
- die Bindung S-CH₂X eine C-anomere Bindung bedeutet,
- X eine Gruppe aus der Reihe -CO-, -CH(OH)-, -CH(NR₁R₂)-, -CHR'-, -C(=CHR')- bedeutet,
- R eine geradkettige oder verzweigte, gesättigte oder ungesättigte Alkyl-, Perfluoralkyl-, Hydrofluoralkylkette, einen Cycloalkyl-, Cycloperfluoralkyl- oder Cyclohydrofluoralkylring mit 1 bis 18 Kohlenstoffatomen, einen Phenyl- oder Benzylrest bedeutet, wobei die Kette, der Ring oder der Rest gegebenenfalls durch ein oder mehrere Heteroatome aus der Reihe Sauerstoff, Schwefel, Stickstoff, Silizium unterbrochen und gegebenenfalls durch mindestens einen Rest aus der Reihe -OR'₁, -SR''₁, -NR'''₁R'₂, -COOR''₂, -CONHR'''₂, -CN, Halogen, Perfluoralkyl, Hydrofluoralkyl und/oder mindestens einen gegebenenfalls substituierten Cycloalkyl-, Aryl- oder Heterocyclusrest substituiert sein können,
- R', R₁, R₂, die gleich oder verschieden sind, der für R angegebenen Definition entsprechen und außerdem ein Wasserstoff und einen Hydroxylrest bedeuten können;
R'₁, R'₂, R''₁, R''₂, R'''₁, R'''₂, die gleich oder verschieden sein können, ein Wasserstoffatom, einen Rest aus der Reihe geradkettiger oder verzweigter, gesättigter oder ungesättigter Alkyl-, Hydroxyl-, Perfluoralkyl- und/oder Hydrofluoralkylrest mit 1 bis 30 Kohlenstoffatomen bedeuten,
sowie seiner physiologisch unbedenklichen Salze und seiner optischen Isomere,
oder einer Zusammensetzung, die in einem physiologisch unbedenklichem Medium das C-Glykosidderivat der Formel (I) enthält, zur Stimulation der Synthese von Glykosaminoglykanen, die einen D-Glucosaminrest und/oder N-Acetyl-D-Glucosaminrest enthalten, und/oder von Proteoglykanen, durch die Fibroblasten und/oder Keratinozyten.

2. Verwendung nach dem vorhergehenden Anspruch, **dadurch gekennzeichnet, dass** bei dem C-Glykosidderivat der Formel (I) R eine geradkettige oder verzweigte, gesättigte oder ungesättigte Alkyl-, Perfluoralkyl-oder Hydrofluoralkylkette, einen Cycloalkyl-, Cycloperfluoralkyl- oder Cyclohydrofluoralkylring mit 1 bis 6 Kohlenstoffatomen, einen Phenyl- oder Benzylrest bedeutet, wobei die Kette, der Ring oder der Rest gegebenenfalls durch ein oder mehrere Heteroatome aus der Reihe Sauerstoff, Schwefel, Stickstoff, Silizium unterbrochen und gegebenenfalls durch mindestens einen Rest aus der Reihe -OR'₁, -SR''₁, -NR'''₁R'₂, -COOR''₂, -CONHR'''₂, -CN, Halogen, Perfluoralkyl, Hydrofluoralkyl und/oder mindestens einen gegebenenfalls substituierten Cycloalkyl-, Aryl- oder Heterocyclusrest substituiert sein können.

3. Verwendung nach dem vorhergehenden Anspruch, **dadurch gekennzeichnet, dass** das C-Glykosidderivat der Formel (I) aus den folgenden ausgewählt ist:
- C-β-D-Xylopyranosid-n-propan-2-on;
- C-α-D-Xylopyranosid-n-propan-2-on;
- 1-Phenyl-2-(C-β-D-xylopyranosid)ethan-1-on;
- 1-Phenyl-2-(C-α-D-xylopyranosid)ethan-1-on;
- 1-[2-(3(Hydroxypropylamino)propyl]-C-β-D-xylopyranose;
- 1-[2-(3-Hydroxypropylamino)propyl]-C-α-D-xylopyranose;
- C-β-D-Xylopyranosid-2-hydroxypropan;
- C-α-D-Xylopyranosid-2-hydroxypropan;
- C-β-D-Xylopyranosid-2-aminopropan;
- C-α-D-Xylopyranosid-2-aminopropan;
- C-β-D-Xylopyranosid-2-phenylaminopropan;
- C-a-D-Xylopyranosid-2-phenylaminopropan;
- 3-Methyl-4-(C-β-D-xylopyranosid)buttersäureethyl-ester;
- 3-Methyl-4-(C-α-D-xylopyranosid)buttersäureethyl-ester;
- 6-(C-β-D-Xylopyranosid)-5-ketohexansäure;
- 6-(C-a-D-Xylopyranosid)-5-ketohexansäure;
- 6-(C-β-D-Xylopyranosid)-5-hydroxyhexansäure;
- 6-(C-α-D-Xylopyranosid)-5-hydroxyhexansäure;
- 6-(C-β-D-Xylopyranosid)-5-aminohexansäure;
- 6-(C-α-D-Xylopyranosid)-5-aminohexansäure;
- 6-(C-β-D-Xylopyranosid)-5-phenylaminohexansäure;
- 6-(C-α-D-Xylopyranosid)-5-phenylaminohexansäure;
- 1-(C-β-D-Xylopyranosid)hexan-2,6-diol;
- 1-(C-α-D-Xylopyranosid)hexan-2,6-diol;
- 5-(C-β-D-Xylopyranosid)-4-ketopentansäure;
- 5-(C-α-D-Xylopyranosid)-4-ketopentansäure;
- 5-(C-β-D-Xylopyranosid)-4-hydroxypentansäure;
- 5-(C-α-D-Xylopyranosid)-4-hydroxypentansäure;
- 5-(C-β-D-Xylopyranosid)-4-aminopentansäure;
- 5-(C-α-D-Xylopyranosid)-4-aminopentansäure;
- 5-(C-β-D-Xylopyranosid)-4-phenylaminopentansäure;
- 5-(C-α-D-Xylopyranosid)-4-phenylaminopentansäure;
- 1-(C-β-D-Xylopyranosid)pentan-2,5-diol;
- 1-(C-α-D-Xylopyranosid)pentan-2,5-diol.

4. Verwendung nach einem der vorhergehenden Ansprüche, **dadurch gekennzeichnet, dass** das C-Glykosidderivat der Formel (I) aus den Folgenden ausgewählt ist:
- C-β-D-Xylopyranosid-n-propan-2-on;
- C-α-D-Xylopyranosid-n-propan-2-on;
- 1-Phenyl-2-(C-β-D-xylopyranosid)ethan-1-on;
- 1-Phenyl-2-(C-α-D-xylopyranosid)ethan-1-on;
- 1-[2-(3(Hydroxypropylamino)propyl]-C-β-D-xylopyranose;
- 1-[2-(3-Hydroxypropylamino)propyl]-C-α-D-xylopyranose;
- C-β-D-Xylopyranosid-2-hydroxypropan;
- C-a-D-Xylopyranosid-2-hydroxypropan.

5. Verwendung nach einem der vorhergehenden Ansprüche, **dadurch gekennzeichnet, dass** es sich bei dem C-Glykosidderivat um C-β-D-Xylopyranosid-2-hydroxypropan handelt.

6. Verwendung nach einem der vorhergehenden Ansprüche, **dadurch gekennzeichnet, dass** das C-Glykosidderivat der Formel (I) in der Zusammensetzung in einer Konzentration von 0,00001% bis 25% des Gesamtgewichts der Zusammensetzung vorliegt.

7. Verwendung nach einem der vorhergehenden Ansprüche, **dadurch gekennzeichnet, dass** C-Glykosidderivat der Formel (I) oder die Zusammensetzung für die Stimulation der Synthese von Hyaluronsäure und/oder von haluronsäurehaltigen Proteoglykanen bestimmt ist.

8. Verfahren zur kosmetischen Behandlung der Haut und/oder der Kopfhaut und/oder der Schleimhäute, um einen Beitrag zu mindestens einer der folgenden Aktivitäten zu leisten:
- Kampf gegen die Alterung der Epidermis,
- Aufrechterhalten und/oder Stimulation des Hydratisierungszustands und/oder Kampf gegen das Austrocknen der Haut in Zusammenhang mit einer unzureichenden Wirkung von GAGs, die einen D-Glucosamin- und/oder N-Acetyl-D-Glucosaminrest enthalten, insbesondere Hyaluronsäure,
- Verbesserung der Spannkraft der Haut,
- Aufrechterhaltung oder Wiederherstellung der Geschmeidigkeit und der Elastizität der Haut,
- Verbesserung des Mineralisierungszustands der Epidermis, wodurch die Haut gesunder gemacht wird und ihre Vitalität verbessert wird,
- Erleichterung von Austauschvorgängen zwischen den Zellen,
- Kampf gegen Hautrissen und rissigem Aussehen der
Haut,
**dadurch gekennzeichnet, dass** man auf die Haut und/oder die Kopfhaut und/oder die Schleimhäute eine kosmetische Zusammensetzung appliziert, die in einem physiologisch unbedenklichen Medium ein C-Glykosidderivat der Formel (I) wie in einem der Ansprüche 1 bis 5 definiert enthält, diese mit der Haut und/oder den Schleimhäuten und/oder der Kopfhaut in Kontakt belässt und gegebenenfalls ausspült.

9. Verfahren nach dem vorhergehenden Anspruch, **dadurch gekennzeichnet, dass** das C-Glykosidderivat der Formel (I) in der Zusammensetzung in einer Konzentration von 0,00001% bis 25% des Gesamtgewichts der Zusammensetzung vorliegt.

10. Verwendung einer Zusammensetzung, die in einem physiologisch unbedenklichen Medium ein C-Glykosidderivat der Formel (I) wie in einem der Ansprüche 1 bis 5 definiert enthält, zur kosmetischen Behandlung von Keratinmaterialien.

11. Verwendung einer Zusammensetzung, die in einem physiologisch unbedenklichen Medium ein C-Glykosidderivat der Formel (I) wie in einem der Ansprüche 1 bis 5 definiert enthält, für die Verbesserung des Aussehens von Keratinmaterialien.

12. Verwendung nach dem vorhergehenden Anspruch, **dadurch gekennzeichnet, dass** die Keratinmaterialien aus der Reihe Haar, Wimpern, Augenbrauen und Nägel ausgewählt sind.

13. Verwendung einer Zusammensetzung, die in einem physiologisch unbedenklichen Medium ein C-Glykosidderivat der Formel (I) wie in einem der Ansprüche 1 bis 5 definiert enthält, für die Haarpflege.

14. Verwendung nach dem vorhergehenden Anspruch, **dadurch gekennzeichnet, dass** es sich bei der Zusammensetzung um eine Lotion oder ein Gel gegen den Haarausfall handelt.

15. Verwendung nach einem der Ansprüche 10 bis 14, **dadurch gekennzeichnet, dass** das C-Glykosidderivat der Formel (I) in der Zusammensetzung in einer Konzentration von 0,00001% bis 25% des Gesamtgewichts der Zusammensetzung vorliegt.

16. C-Glykosidderivat der folgenden Formel (II): 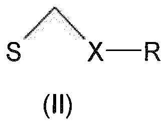 in der
- S D-Xylose bedeutet,
- die Bindung S-CH₂X eine C-anomere Bindung bedeutet,
- X eine Gruppe aus der Reihe -CH(OH)-, -CH (NR1R2) - bedeutet,
- R eine geradkettige oder verzweigte, gesättigte oder ungesättigte Alkyl-, Perfluoralkyl-, Hydrofluoralkylkette, einen Cycloalkyl-, Cycloperfluoralkyl- oder Cyclohydrofluoralkylring mit 1 bis 18 Kohlenstoffatomen, einen Phenyl- oder Benzylrest bedeutet, wobei die Kette, der Ring oder der Rest gegebenenfalls durch ein oder mehrere Heteroatome aus der Reihe Sauerstoff, Schwefel, Stickstoff, Silizium unterbrochen und gegebenenfalls durch mindestens einen Rest aus der Reihe -OR'₁, -SR''₁, -NR'''₁R'₂, -COOR''₂, -CONHR'''₂, -CN, Halogen, Perfluoralkyl, Hydrofluoralkyl und/oder mindestens einen gegebenenfalls substituierten Cycloalkyl-, Aryl- oder Heterocyclusrest substituiert sein können,
- R₁, R₂, die gleich oder verschieden sind, der für R angegebenen Definition entsprechen und außerdem ein Wasserstoff und einen Hydroxylrest bedeuten können;
R'₁, R'₂, R''₁, R''₂, R'''₁, R'''₂, die gleich oder verschieden sein können, ein Wasserstoffatom, einen Rest aus der Reihe geradkettiger oder verzweigter, gesättigter oder ungesättigter Alkyl-, Hydroxyl-, Perfluoralkyl- und/oder Hydrofluoralkylrest mit 1 bis 30 Kohlenstoffatomen bedeuten,
sowie seine physiologisch unbedenklichen Salze und seine optischen Isomere.

17. C-Glykosidderivat nach dem vorhergehenden Anspruch, **dadurch gekennzeichnet, dass** R eine geradkettige oder verzweigte, gesättigte oder ungesättigte Alkyl-, Perfluoralkyl- oder Hydrofluoralkylkette, einen Cycloalkyl-, Cycloperfluoralkyl oder Cyclohydrofluoralkylring mit 1 bis 6 Kohlenstoffatomen, einen Phenyl- oder Benzylrest bedeutet, wobei die Kette, der Ring oder der Rest gegebenenfalls durch ein oder mehrere Heteroatome aus der Reihe Sauerstoff, Schwefel, Stickstoff, Silizium unterbrochen und gegebenenfalls durch mindestens einen Rest aus der Reihe -OR'₁, -SR''₁, -NR'''₁R'₂, -COOR''₂, -CONHR'''₂, -CN, Halogen, Perfluoralkyl, Hydrofluoralkyl und/oder mindestens einen gegebenenfalls substituierten Cycloalkyl-, Aryl- oder Heterocyclusrest substituiert sein können.

18. C-Glykosidderivat nach einem der Ansprüche 16 oder 17, **dadurch gekennzeichnet, dass** es aus der Reihe
- 1-[2-(3-Hydroxypropylamino)propyl]-C-β-D-xylopyranose;
- 1-[2-(3-Hydroxypropylamino)propyl]-C-a-D-xylopyranose;
- C-β-D-Xylopyranosid-2-hydroxypropan;
- C-α-D-Xylopyranosid-2-hydroxypropan;
- C-β-D-Xylopyranosid-2-aminopropan;
- C-a-D-Xylopyranosid-2-aminopropan;
- C-β-D-Xylopyranosid-2-phenylaminopropan;
- C-α-D-Xylopyranosid-2-phenylaminopropan;
- 6-(C-β-D-Xylopyranosid)-5-hydroxyhexansäure;
- 6-(C-α-D-Xylopyranosid)-5-hydroxyhexansäure;
- 6-(C-β-D-Xylopyranosid)-5-aminohexansäure;
- 6-(C-α-D-Xylopyranosid)-5-aminohexansäure;
- 6-(C-β-D-Xylopyranosid)-5-phenylaminohexansäure;
- 6-(C-α-D-Xylopyranosid)-5-phenylaminohexansäure;
- 5-(C-β-D-Xylopyranosid)-4-hydroxypentansäure;
- 5-(C-α-D-Xylopyranosid)-4-hydroxypentansäure;
- 5-(C-β-D-Xylopyranosid)-4-aminopentansäure;
- 5-(C-α-D-Xylopyranosid)-4-aminopentansäure;
- 5-(C-β-D-Xylopyranosid)-4-phenylaminopentansäure;
- 5-(C-α-D-Xylopyranosid)-4-phenylaminopentansäure ausgewählt ist.

19. C-Glykosidderivat nach einem der Ansprüche 16 bis 18, **dadurch gekennzeichnet, dass** es aus der Reihe
- 1-[2-(3-Hydroxypropylamino)propyl]-C-β-D-xylopyranose;
- 1-[2-(3-Hydroxypropylamino)propyl]-C-α-D-xylopyranose;
- C-β-D-Xylopyranosid-2-hydroxypropan;
- C-α-D-Xylopyranosid-2-hydroxypropan ausgewählt ist.

20. C-Glykosidderivat nach einem der Ansprüche 16 bis 19, **dadurch gekennzeichnet, dass** es sich um C-β-D-Xylopyranosid-2-hydroxypropan handelt.

21. Zusammensetzung, die ein C-Glykosidderivat nach einem der Ansprüche 16 bis 20 in einem physiologisch unbedenklichen Medium umfasst.

22. Zusammensetzung nach dem vorhergehenden Anspruch, **dadurch gekennzeichnet, dass** das C-Glykosidderivat in einer Konzentration von 0,00001% bis 25% des Gesamtgewichts der Zusammensetzung vorliegt.

23. Zusammensetzung nach einem der Ansprüche 21 oder 22, **dadurch gekennzeichnet, dass** sie einen kosmetischen Hilfsstoff aus der Reihe hydrophile oder lipophile Geliermittel, hydrophile oder lipophile Wirkstoffe, Konservierungsmittel, Antioxidationsmittel, Lösungsmittel, Duftstoffe, Füllstoffe, Filter, Pigmente, Chelatbildner, Geruchsabsorptionsmittel und Farbmittel umfasst.

24. Zusammensetzung nach einem der Ansprüche 21 bis 23, **dadurch gekennzeichnet, dass** sie in Form einer Emulsion vorliegt.

## Claims

1. Cosmetic use of a C-glycoside derivative corresponding to formula (I) below: 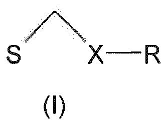 in which,
- S represents D-xylose,
- the linkage S-CH₂X represents a linkage of C-anomeric nature,
- X represents a group chosen from: -CO-, -CH (OH) -, -CH(NR₁R₂)-, -CHR'- and -C(=CHR')-,
- R represents a saturated or unsaturated, linear or branched alkyl, perfluoroalkyl or hydrofluoroalkyl chain, or a cycloalkyl, cycloperfluoroalkyl or cyclohydrofluoroalkyl ring, comprising from 1 to 18 carbon atoms, a phenyl radical or a benzyl radical, it being possible for said chain, said ring or said radical to be optionally interrupted with one or more heteroatoms chosen from oxygen, sulfur, nitrogen and silicon, and optionally substituted with at least one radical chosen from -OR'₁, -SR''₁, -NR'''₁R'₂, -COOR''₂, -CONHR'''2, -CN, halogen, perfluoroalkyl and hydrofluoroalkyl and/or at least one cycloalkyl, aryl or heterocyclic radical, which are optionally substituted,
- R', R₁ and R₂, which may be identical or different, have the same definition as that given for R, and can also represent a hydrogen and a hydroxyl radical,
- R'₁, R'₂, R''₁, R''₂, R'''₁ and R'''₂, which may be identical or different, represent a hydrogen atom or a radical chosen from a saturated or unsaturated, linear or branched alkyl, hydroxyl, perfluoroalkyl and/or hydrofluoroalkyl radical comprising from 1 to 30 carbon atoms,
and also the physiologically acceptable salts thereof and the optical isomers thereof,
or of a composition comprising, in a physiologically acceptable medium, said C-glycoside derivative of formula (I), for stimulating the synthesis of glycosaminoglycans containing a D-glucosamine and/or N-acetyl-D-glucosamine residue, and/or proteoglycans, by fibroblasts and/or keratino-cytes.

2. Use according to the preceding claim, **characterized in that**, for the C-glycoside derivative of formula (I), R represents a saturated or unsaturated, linear or branched alkyl, perfluoroalkyl or hydrofluoroalkyl chain, or a cycloalkyl, cycloperfluoroalkyl or cyclohydrofluoroalkyl ring, comprising from 1 to 6 carbon atoms, a phenyl radical or a benzyl radical, it being possible for said chain, said ring or said radical to be optionally interrupted with one or more heteroatoms chosen from oxygen, sulfur, nitrogen and silicon, and optionally substituted with at least one radical chosen from -OR'₁, -SR''₁, -NR'''₁R'₂, -COOR''₂, -CONHR'''₂, -CN, halogen, perfluoroalkyl and hydrofluoroalkyl and/or at least one cycloalkyl, aryl or heterocyclic radical, which are optionally substituted.

3. Use according to the preceding claim, **characterized in that** the C-glycoside derivative of formula (I) is chosen from:
- C-β-D-xylopyranoside-n-propan-2-one;
- C-α-D-xylopyranoside-n-propan-2-one;
- 1-phenyl-2-(C-β-D-xylopyranoside)ethan-1-one;
- 1-phenyl-2-(C-α-D-xylopyranoside)ethan-1-one;
- 1-[2-(3-hydroxypropylamino)propyl]-C-β-D-xylo-pyranose;
- 1-[2-(3-hydroxypropylamino)propyl]-C-α-D-xylo-pyranose;
- C-β-D-xylopyranoside-2-hydroxypropane;
- C-α-D-xylopyranoside-2-hydroxypropane;
- C-β-D-xylopyranoside-2-aminopropane;
- C-α-D-xylopyranoside-2-aminopropane;
- C-β-D-xylopyranoside-2-phenylaminopropane;
- C-α-D-xylopyranoside-2-phenylaminopropane;
- 3-methyl-4-(C-β-D-xylopyranoside)butyric acid ethyl ester;
- 3-methyl-4-(C-α-D-xylopyranoside)butyric acid ethyl ester;
- 6-(C-β-D-xylopyranoside)-5-ketohexanoic acid;
- 6-(C-α-D-xylopyranoside)-5-ketohexanoic acid;
- 6-(C-β-D-xylopyranoside)-5-hydroxyhexanoic acid;
- 6-(C-α-D-xylopyranoside)-5-hydroxyhexanoic acid;
- 6-(C-β-D-xylopyranoside)-5-aminohexanoic acid;
- 6-(C-α-D-xylopyranoside)-5-aminohexanoic acid;
- 6-(C-β-D-xylopyranoside)-5-phenylaminohexanoic acid;
- 6-(C-α-D-xylopyranoside)-5-phenylaminohexanoic acid;
- 1-(C-β-D-xylopyranoside)hexane-2,6-diol;
- 1-(C-α-D-xylopyranoside)hexane-2,6-diol;
- 5-(C-β-D-xylopyranoside)-4-ketopentanoic acid;
- 5-(C-α-D-xylopyranoside)-4-ketopentanoic acid;
- 5-(C-β-D-xylopyranoside)-4-hydroxypentanoic acid;
- 5-(C-α-D-xylopyranoside)-4-hydroxypentanoic acid;
- 5-(C-β-D-xylopyranoside)-4-aminopentanoic acid;
- 5-(C-α-D-xylopyranoside)-4-aminopentanoic acid;
- 5-(C-β-D-xylopyranoside)-4-phenylaminopentanoic acid;
- 5-(C-α-D-xylopyranoside)-4-phenylaminopentanoic acid;
- 1-(C-β-D-xylopyranoside)pentane-2,5-diol;
- 1-(C-α-D-xylopyranoside)pentane-2,5-diol.

4. Use according to any one of the preceding claims, **characterized in that** the C-glycoside derivative of formula (I) is chosen from:
- C-β-D-xylopyranoside-n-propan-2-one;
- C-α-D-xylopyranoside-n-propan-2-one;
- 1-phenyl-2-(C-β-D-xylopyranoside)ethan-1-one;
- 1-phenyl-2-(C-α-D-xylopyranoside)ethan-1-one;
- 1-[2-(3-hydroxypropylamino)propyl]-C-β-D-xylo-pyranose;
- 1-[2-(3-hydroxypropylamino)propyl]-C-α-D-xylo-pyranose;
- C-β-D-xylopyranoside-2-hydroxypropane;
- C-α-D-xylopyranoside-2-hydroxypropane.

5. Use according to any one of the preceding claims, **characterized in that** the C-glycoside derivative is C-β-D-xylopyranoside-2-hydroxypropane.

6. Use according to any one of the preceding claims, **characterized in that** the C-glycoside derivative of formula (I) is present in the composition in a content ranging from 0.00001% to 25% of the total weight of the composition.

7. Use according to any one of the preceding claims, **characterized in that** the C-glycoside derivative of formula (I) or the composition is intended for stimulating the synthesis of hyaluronic acid and/or of proteoglycans containing hyaluronic acid.

8. Cosmetic treatment process for the skin and/or the scalp and/or the mucous membranes for contributing to at least one of the following activities:
- combating ageing of the epidermis,
- maintaining and/or stimulating moisturization and/or combating drying-out of the skin associated with insufficiency of the action of GAGs containing a D-glucosamine and/or N-acetyl-D-glucosamine residue, in particular hyaluronic acid,
- improving the tonicity of the skin,
- maintaining or restoring the suppleness and the elasticity of the skin,
- improving the mineralization of the epidermis, thus making the skin healthier and improving its vitality,
- facilitating intercellular exchanges,
- combating chapping and the cracked appearance of the skin,
**characterized in that** a cosmetic composition comprising, in a physiologically acceptable medium, a C-glycoside derivative of formula (I) as defined according to any one of Claims 1 to 5 is applied to the skin and/or the scalp and/or the mucous membranes, it is left in contact with the skin and/or the mucous membranes and/or the scalp, and optionally rinsed off.

9. Process according to the preceding claim, **characterized in that** the C-glycoside derivative of formula (I) is present in the composition in a content ranging from 0.00001% to 25% of the total weight of the composition.

10. Use of a composition comprising, in a physiologically acceptable medium, a C-glycoside derivative of formula (I) as defined according to any one of Claims 1 to 5, for the cosmetic treatment of keratin materials.

11. Use of a composition comprising, in a physiologically acceptable medium, a C-glycoside derivative of formula (I) as defined according to any one of Claims 1 to 5, for improving the appearance of keratin materials.

12. Use according to the preceding claim, **characterized in that** the keratin materials are chosen from the hair, the eyelashes, the eyebrows and the nails.

13. Use of a composition comprising, in a physiologically acceptable medium, a C-glycoside derivative of formula (I) as defined according to any one of Claims 1 to 5, for hair care.

14. Use according to the preceding claim, **characterized in that** the composition is a lotion or gel for preventing hair loss.

15. Use according to any one of Claims 10 to 14, **characterized in that** the C-glycoside derivative of formula (I) is present in the composition in a content ranging from 0.00001% to 25% of the total weight of the composition.

16. C-Glycoside derivative corresponding to formula (II) below: 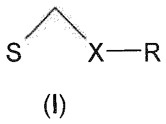 in which,
- S represents D-xylose,
- the linkage S-CH₂X represents a linkage of C-anomeric nature,
- X represents a group chosen from: -CH(OH)- and -CH(NR₁R₂)-,
- R represents a saturated or unsaturated, linear or branched alkyl, perfluoroalkyl or hydrofluoroalkyl chain, or a cycloalkyl, cycloperfluoroalkyl or cyclohydrofluoroalkyl ring, comprising from 1 to 18 carbon atoms, a phenyl radical or a benzyl radical, it being possible for said chain, said ring or said radical to be optionally interrupted with one or more heteroatoms chosen from oxygen, sulfur, nitrogen and silicon, and optionally substituted with at least one radical chosen from -OR'₁, -SR''₁, -NR'''₁R'₂, -COOR''₂, -CONHR'''₂, -CN, halogen, perfluoroalkyl and hydrofluoroalkyl and/or at least one cycloalkyl, aryl or heterocyclic radical, which are optionally substituted,
- R₁ and R₂, which may be identical or different, have the same definition as that given for R, and can also represent a hydrogen and a hydroxyl radical,
- R'₁, R'₂, R''₁, R''₂, R'''₁ and R'''₂, which may be identical or different, represent a hydrogen atom or a radical chosen from a saturated or unsaturated, linear or branched alkyl, hydroxyl, perfluoroalkyl and/or hydrofluoroalkyl radical comprising from 1 to 30 carbon atoms,
and also the physiologically acceptable salts thereof and the optical isomers thereof.

17. C-Glycoside derivative according to the preceding claim, **characterized in that** R represents a saturated or unsaturated, linear or branched alkyl, perfluoroalkyl or hydrofluoroalkyl chain, or a cycloalkyl, cycloperfluoroalkyl or cyclohydrofluoroalkyl ring, comprising from 1 to 6 carbon atoms, a phenyl radical or a benzyl radical, it being possible for said chain, said ring or said radical to be optionally interrupted with one or more heteroatoms chosen from oxygen, sulfur, nitrogen and silicon, and optionally substituted with at least one radical chosen from -OR'₁, -SR''₁, -NR'''₁R'₂, -COOR''₂, -CONHR'''₂, -CN, halogen, perfluoroalkyl and hydrofluoroalkyl and/or at least one cycloalkyl, aryl or heterocyclic radical, which are optionally substituted.

18. C-Glycoside derivative according to either one of Claims 16 and 17, **characterized in that** it is chosen from:
- 1-[2-(3-hydroxypropylamino)propyl]-C-β-D-xylo-pyranose;
- 1-[2-(3-hydroxypropylamino)propyl]-C-α-D-xylo-pyranose;
- C-β-D-xylopyranoside-2-hydroxypropane;
- C-α-D-xylopyranoside-2-hydroxypropane;
- C-β-D-xylopyranoside-2-aminopropane;
- C-α-D-xylopyranoside-2-aminopropane;
- C-β-D-xylopyranoside-2-phenylaminopropane;
- C-α-D-xylopyranoside-2-phenylaminopropane;
- 6-(C-β-D-xylopyranoside)-5-hydroxyhexanoic acid;
- 6-(C-α-D-xylopyranoside)-5-hydroxyhexanoic acid;
- 6-(C-β-D-xylopyranoside)-5-aminohexanoic acid;
- 6-(C-α-D-xylopyranoside)-5-aminohexanoic acid;
- 6-(C-β-D-xylopyranoside)-5-phenylaminohexanoic acid;
- 6-(C-α-D-xylopyranoside)-5-phenylaminohexanoic acid;
- 5-(C-β-D-xylopyranoside)-4-hydroxypentanoic acid;
- 5-(C-α-D-xylopyranoside)-4-hydroxypentanoic acid;
- 5-(C-β-D-xylopyranoside)-4-aminopentanoic acid;
- 5-(C-α-D-xylopyranoside)-4-aminopentanoic acid;
- 5-(C-β-D-xylopyranoside)-4-phenylaminopentanoic acid;
- 5-(C-α-D-xylopyranoside)-4-phenylaminopentanoic acid.

19. C-Glycoside derivative according to any one of Claims 16 to 18, **characterized in that** it is chosen from:
- 1-[2-(3-hydroxypropylamino)propyl]-C-β-D-xylo-pyranose;
- 1-[2-(3-hydroxypropylamino)propyl]-C-a-D-xylo-pyranose;
- C-β-D-xylopyranoside-2-hydroxypropane;
- C-α-D-xylopyranoside-2-hydroxypropane.

20. C-Glycoside derivative according to any one of Claims 16 to 19, **characterized in that** it is C-β-D-xylopyranoside-2-hydroxypropane.

21. Composition comprising, in a physiologically acceptable medium, a C-glycoside derivative according to any one of Claims 16 to 20.

22. Composition according to the preceding claim, **characterized in that** the C-glycoside derivative is present in a content ranging from 0.00001% to 25% of the total weight of the composition.

23. Composition according to either of Claims 21 and 22, **characterized in that** it comprises a cosmetic adjuvant chosen from hydrophilic or lipophilic gelling agents, hydrophilic or lipophilic active agents, preservatives, antioxidants, solvents, fragrances, fillers, screening agents, pigments, chelating agents, odour absorbers and colorants.

24. Composition according to one of Claims 21 to 23, **characterized in that** it is in the form of an emulsion.
